# EUROPÄISCHE PATENTSCHRIFT

(11) **EP 0 906 303 B1**
(45) Veröffentlichungstag und Bekanntmachung des Hinweises auf die Patenterteilung: **09.04.2003**
(21) Anmeldenummer: 97928221.7
(22) Anmeldetag: 18.06.1997
(51) Int. Cl.: C07D 311/74, C07D 493/04, C07D 491/153, A61K 31/35, A61K 31/505

(54) **RINGANNELIERTE DIHYDROPYRANE, VERFAHREN ZUR HERSTELLUNG SOWIE DEREN VERWENDUNG**
RING-FUSED DIHYDROPYRANES, PROCESS FOR THE PREPARATION AND USE THEREOF
DIHYDROPRANNES ANNELES CYCLIQUES, LEUR PROCEDE DE FABRICATION ET LEUR UTILISATION

(30) Priorität: 18.06.1996 DE 19624154
(43) Veröffentlichungstag der Anmeldung: 07.04.1999
(73) Patentinhaber: HOECHST AKTIENGESELLSCHAFT, 65929 Frankfurt am Main (DE)
(72) Erfinder: HENKE, Stephan, D-65719 Hofheim (DE)
(86) Internationale Anmeldenummer: EP9703146
(87) Internationale Veröffentlichungsnummer: WO97048691

(56) Entgegenhaltungen:
- US-A- 3 775 435

## Beschreibung

Die Erfindung betrifft Verbindungen der Formel I 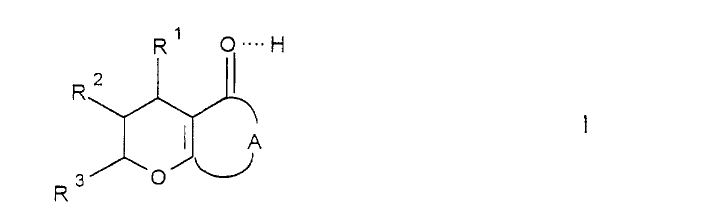 in welcher bedeuten:
- R¹: 1. (C₁-C₁₄)-Alkyl,
2. (C₂-C₆)-Alkenyl,
3. (C₀-C₆)-Alkyl-(C₃-C₁₀)-cycloalkyl-(C₀-C₆)-alkyl, wobei der Alkylteil gegebenfalls mit einer oder mehreren OH-Gruppen substituiert ist,
4. (C₀-C₆)-Alkyl-(C₆-C₁₄)-Aryl,
5. (C₀-C₆)-Alkyl-(C₃-C₉)-Heteroaryl,
6. (C₂-C₆)-Alkenyl-(C₆-C₁₄)-Aryl,
7. (C₂-C₆)-Alkenyl-(C₃-C₉)-Heteroaryl,
8. (C₁-C₆)-Alkanoyl,
9. einen wie unter 4.,5.,6. oder 7. definierten Rest, wobei der (C₆-C₁₄)-Aryl-, bzw. (C₃-C₉)-Heteroarylteil mit 1, 2 oder 3 gleichen oder verschiedenen Resten aus der Reihe (C₁-C₁₀)-Alkyl, Carboxy, Amino, Nitro, (C₁-C₄)-Alkylamino, Hydroxy; (C₁-C₄)-Alkoxy, wobei ein bis alle Wasserstoff-Atome durch Fluoratome ersetzt sein können, (C₆-C₁₂)-Aryloxy, Halogen, Cyano, Di-(C₁-C₄)-Alkylamino, Carbamoyl, Sulfamoyl und (C₁-C₄)-Alkoxycarbonyl substituiert ist, oder zwei benachbarte Reste am (C₆-C₁₂)-Arylring zusammen Alkylendioxy, vorzugsweise Methylendioxy bedeuten;
- R²: Wasserstoff, (C₁-C₁₀)-Alkyl, (C₀-C₆)-Alkyl-(C₆-C₁₂)-Aryl (C₀-C₆)-Alkyl-(C₃-C₉)-Heteroaryl, wobei der C₆-C₁₂-Aryl-, bzw. C₃-C₉-Heteroarylteil gegebenenfalls durch 1, 2 oder 3 gleiche oder verschiedene Reste aus der Reihe Carboxy, Amino, (C₁-C₄)-Alkylamino, Hydroxy, (C₁-C₄)-Alkoxy, Halogen, Cyano, Di-(C₁-C₄)-alkylamino, Carbamoyl, Sulfamoyl und (C₁-C₄)-Alkoxycarbonyl substituiert ist;
- R³: -X-R⁴;
- X: -O-, -NR⁵-, oder -S-;
- R⁴: wie R¹ definiert ist;
wobei R⁴ zusammen mit R² ein Brückenglied -CH₂-CH₂- oder -CH₂-CH₂-CH₂- bilden kann;
- R⁵: Wasserstoff oder (C₁-C₆)-Alkyl bedeutet;
- A: ein anneliertes zyklisches Ringsystem bedeutet, das
a) mit einer, zwei oder drei, vorzugsweise mit einer oder zwei Oxo- oder Hydroxyfunktionen substituiert ist, wobei eine Hydroxy- oder Oxo-Funktion in Nachbarstellung zum Dihydropyranring steht, und
b) ein-, zwei- oder mehrfach, vorzugsweise ein- oder zweifach mit einem (C₁-C₁₀) Alkyl-Rest oder einer Carboxylgruppe substituiert ist, wobei zumindest ein Alkylsubstituent eine funktionelle Gruppe, wie Hydroxy, Carboxy oder Amino trägt;
sowie deren physiologisch verträglichen Salze.

Beispiele für annelierte zyklische Ringsysteme A sind 5- oder 6-Ring-Strukturen, die aromatisch oder nichtaromatisch sein können und im Ringgerüst Sauerstoff oder Stickstoff tragen können. Insbesondere eignen sich Cyclopentan, Cyclohexan, Tetrahydrofuran, Benzol, Pyridin, Imidazol, Pyrazol, Piperazin, Dioxan und Pyrimidin.

Unter (C₆-C₁₄)-Aryl wird beispielsweise Phenyl, Naphtyl, Anthracenyl oder Biphenyl verstanden.
Alkyl und davon abgeleitete Reste wie Alkoxy können geradkettig oder verzweigt sein. Halogen steht vorzugsweise für Fluor, Chlor oder Brom.

Ein Heteroaryl-Rest im Sinne der vorliegenden Erfindung ist der Rest eines monocyclischen oder bicyclischen (C₃-C₉)-Heteroaromaten, der im Ringsystem ein oder zwei N-Atome und/oder ein S- oder ein O-Atom enthält. Zum Begriff "Heteroaromat" siehe Garrat, Vollhardt, Aromatizität, Stuttgart 1973, Seiten 131-153. Beispiele geeigneter Heteroarylreste sind die Reste von Thiophen, Furan, Benzo[b]thiophen, Benzofuran, Pyrrol, Imidazol, Pyrazol, Pyridin, Pyrazin, Pyrimidin,

Pyridazin, Indol, Chinolin, Isochinolin, Oxazol, Isoxazol, Thiazol, Isothiazol, Isobenzofuran, Indolizin, Isoindol, Indazol, Phthalazin, Naphthyridin, Chinoxalin, Chinazolin, Cinnolin und Furazan.

Aryl, Alkyl, Heteroaryl und davon abgeleitete Reste können wie oben angegeben einfach oder, falls chemisch möglich, auch mehrfach substituiert sein.

Funktionelle Gruppen wie Hydroxy, Carboxy oder Amino können auch mit einer üblichen chemischen Schutzgruppe versehen sein.

Chiralitätszentren können, wenn nicht anders angegeben, in der R- oder in der S-Konfiguration vorliegen. Die Erfindung betrifft sowohl die optisch reinen Verbindungen als auch Stereoisomerengemische wie Enantiomerengemische und Diasteromerengemische.

Die am anellierten Ringsystem A befindlichen Hydroxy- bzw. Oxogruppen können in allen tautomeren Formen vorliegen.

Als Salze kommen insbesondere Alkali- und Erdalkalisalze, Salze mit physiologisch verträglichen Aminen und Salze mit anorganischen oder organischen Säuren wie z.B. HCl, HBr, H₂SO₄, Maleinsäure, Fumarsäure in Frage.

Bei den oben beschriebenen Verbindungen der Formel I handelt es sich um Derivate des Dihydropyrans, die mit Hilfe kombinatorischer Methoden schnell, automatisiert und in guten Ausbeuten am festen Trägermaterial (FestphasenSynthese) synthetisiert werden können.

Die oben beschriebenen Verbindungen besitzen eine pharmakologische Wirkung auf verschiedene Erkrankungsformen, wie Stoffwechselerkrankungen, z.B. Diabetes und Arteriosklerose, auf Erkrankungen des Herz-Kreislaufs- und des zentralen Nervensystems sowie auf Erkrankungen des Knochenstoffwechsels. Sie haben immunmodulierende Eigenschaften und sind zur Behandlung von Krebs sowie Autoimmunerkrankungen geeignet. Darüber hinaus ist eine antiinfektive Wirkung zu beobachten.

Bevorzugt sind Verbindungen der Formel I in denen
- R¹: (C₁-C₈)-Alkyl, (C₀-C₆)-Alkyl-(C₆-C₁₂)-aryl, (C₀-C₆)-Alkyl-(C₃-C₉)-Heteroaryl oder (C₀-C₆)-Alkyl-(C₆-C₁₂)-aryl, wobei der (C₆-C12)-Arylteil mit 1, 2 oder 3 gleichen oder verschiedenen Resten aus der Reihe (C₁-C₆)-Alkyl, Carboxy, Amino, Nitro, Hydroxy, (C₁-C₆)-Alkoxy, Halogen substituiert ist oder zwei benachbarte Reste am (C₆-C₁₂)-Arylring zusammen Methylendioxy bedeuten; bedeutet;
- R²: Wasserstoff oder (C₁-C₆)-Alkyl, bevorzugt Wasserstoff, bedeutet;
- R³: -X-R⁴ ist;
- X: -O-, NR5 oder -S- bedeutet;
- R⁴: (C₁-C₆)-Alkyl, das gegebenfalls mit einem oder zwei Resten aus der Reihe OH, NR⁵ substituiert ist; (C₀-C₆)-Alkyl-(C₆-C₁₂)-aryl, vorzugsweise Phenyl oder Benzyl; (C₃-C₆)-Cycloalkyl oder (C₁-C₆)-Alkanoyl bedeutet und
wobei R⁴ zusammen mit R² ein Brückenglied -CH₂-CH₂- oder -CH₂-CH₂-CH₂- bilden kann;
- R⁵: Wasserstoff oder (C₁-C₆)-Alkyl bedeutet; sowie deren physiologisch verträglichen Salze.

Bevorzugt sind ferner Verbindungen der Formel I in denen R¹ (C₁-C₆)-Alkyl, (C₀-C₆)-Alkyl-(C₆-C₁₂)-Aryl, beispielsweise Phenyl oder Benzyl bedeuten. Bevorzugt sind auch Verbindungen der Formel 1, in denen R² Wasserstoff oder (C₁-C₆)-Alkyl bedeutet, sowie Verbindungen der Formel I, in denen X gleich -O- ist.

Bevorzugt sind ferner Verbindungen der Formel I, in denen das annelierte zyklische Ringsystem A 1,3-Pyrimidin, Benzol, Dioxan oder Cyclohexan bedeutet.

Die Synthese erfolgt allgemein durch eine geeignete Anbindung von zyklischen 1,3-Dicarbonylverbindungen über ein chemisches Bindeglied (Linker) an eine polymere Matrix nach dem Fachmann bekannten Methoden. Als polymere Matrix eignen sich z.B. Polystyrol, Polytetrafluorethylen, Polyacrylamid, die ggf. mit Polyoxyethylenketten (Spacer) zur Verbesserung der Quellbarkeit verlängert sein können. Als Linker-Einheit eignen sich Strukturen, die spezifisch durch Säure, Base, Reduktion, Oxidation, durch Licht oder mit Fluoridionen die synthetisierte Verbindung freisetzen, wobei die Linker-Einheit an der polymeren Matrix verbleibt (für eine Übersicht über Linkergruppen in der Festphasensynthese siehe J. Früchtel, G. Jung, Angew. Chemie Int. Ed. 1996, 35, 17-42).

Die solchermaßen über eine geeignete funktionelle Gruppe, wie -COOH, -OH, -NH₂ an das Polymer geknüpften 1,3-Dicarbonylverbindungen lassen sich weiteren üblichen Reaktionen der organischen Chemie zuführen. Die Synthese an der festen Phase hat dabei den Vorteil, daß Reagenzien und Reaktanten im großen Überschuß angewendet werden können, Lösungsmittel weit variiert werden können und eine Reinigung durch einfaches Waschen der Harzpartikel erfolgt. Nach sequentieller Durchführung der Synthesestufen erfolgt eine Abspaltung der neu synthetisierten Verbindungen mit Hilfe spezifischer Reagenzien, entsprechend der Linkerauswahl (für eine Beschreibung der Festphasensynthese siehe: J. Früchtel, G. Jung Angew. Chemie Int. Ed. 1996, 35, 17-42).

Die Festphasensynthese von Ring annelierten Dihydropyranstrukturen ist mittels einer Tandem-Knoevenagel-Diels-Alder-Reaktion (L.F. Tietze et al, J. Heterocyclic Chemistry 1990, 27, 47 f) durchführbar (Schema 1; Li = Linker, P = polymere Matrix).

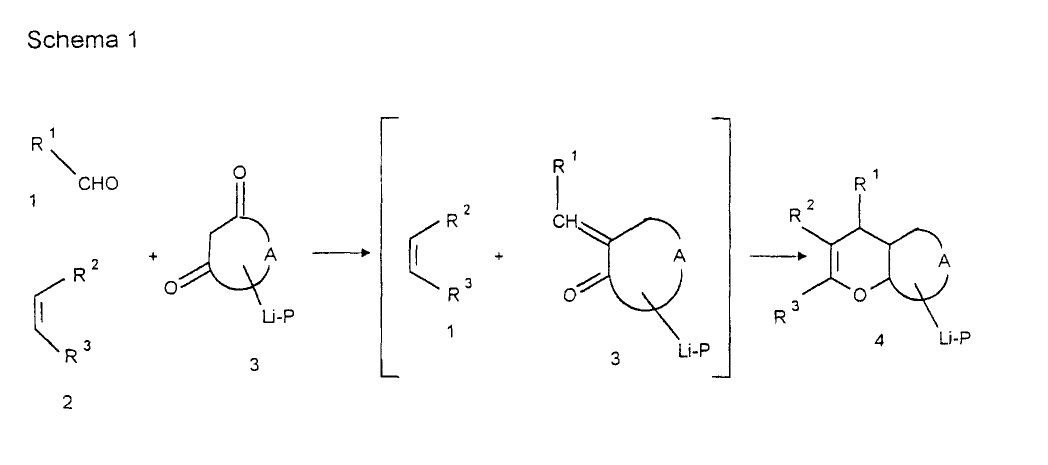

### Herstellungsverfahren:

### A. Synthese von polymer gebundenem 1,3-Dioxocyclohexan-5carbonsäure

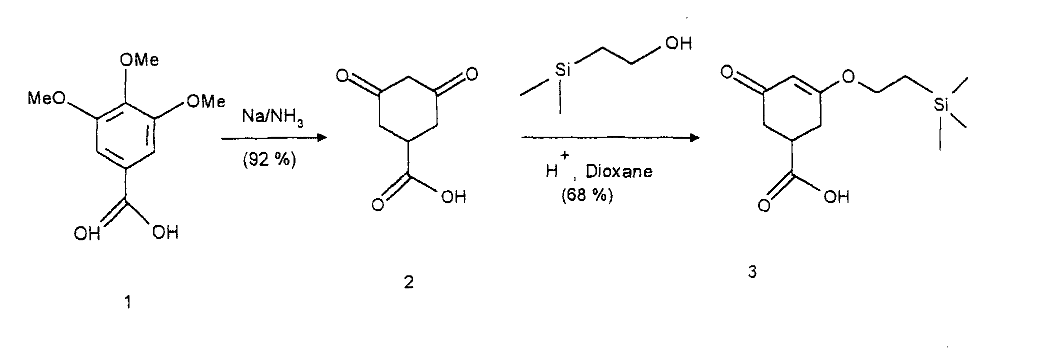

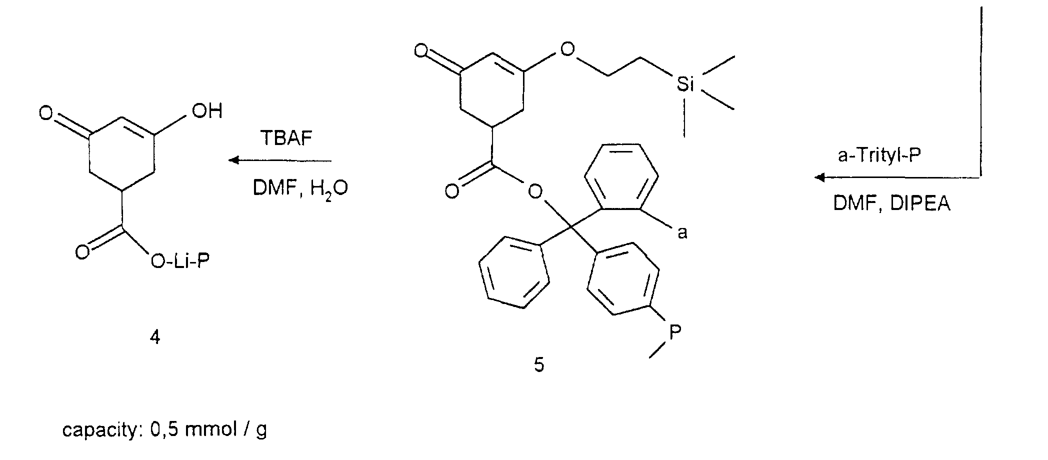

### 1. Synthese von (3)

60 g des literaturbekannten Diketons (2) werden in 300 ml Dioxan abs gelöst und 1 ml konz. Schwefelsäure zugefügt. Man addiert 54 ml 2-Trimethylsilylethanol und läßt 24 h bei Raumtemperatur reagieren. Nach Chromatographie an Kieselgel werden 38 g von (3) erhalten.

### 2. Synthese von (5)

50 g käufliches 2-Chlortrityl-Polystyrolharz (Fa. Novabiochem), das mit 2% Divinylbenzol quervernetzt ist, werden in 300 ml DMF aufgeschlemmt und über 10 min zum Quellen gebracht. Man addiert 11.0 g (3) und 14.4 ml N-Ethylmorpholin. Nach 18 h bei Raumtemperatur wird die Lösung über eine Fritte vom Harz abgetrennt und das Harz gründlich mit DMF und Methylenchlorid gewaschen. Das Harz wird mit Methanol über 10 min inkubiert und erneut gründlich mit DMF und Methylenchlorid gewaschen. Man erhält 60 g (5).

### 3. Synthese von (4)

50 g (5) werden mit 16.5 g Tetrabutylammoniumfluorid (TBAF) und 300 ml DMF abs. versetzt und über 4 h bei Raumtemperatur geschüttelt. Die Lösung wird über eine Fritte abgesaugt und das Harz mit DMF und Methylenchlorid gründlich gewaschen. Nach Trocknen im Vakuum werden 57 g (4) erhalten. Eine Probeabspaltung mit Essigsäure liefert (2) in 95 %iger Reinheit (HPLC,MS). Die Harzbeladung wird mit 0.5 mmol/g ermittelt.

### B. Synthese der Cyclohexan und Benzol annelierten Dihydropyrane (Schema 2)

hier beispielhaft für: R¹ =n-Hexyl, R²= H, R³= - O-tert.-Butyl

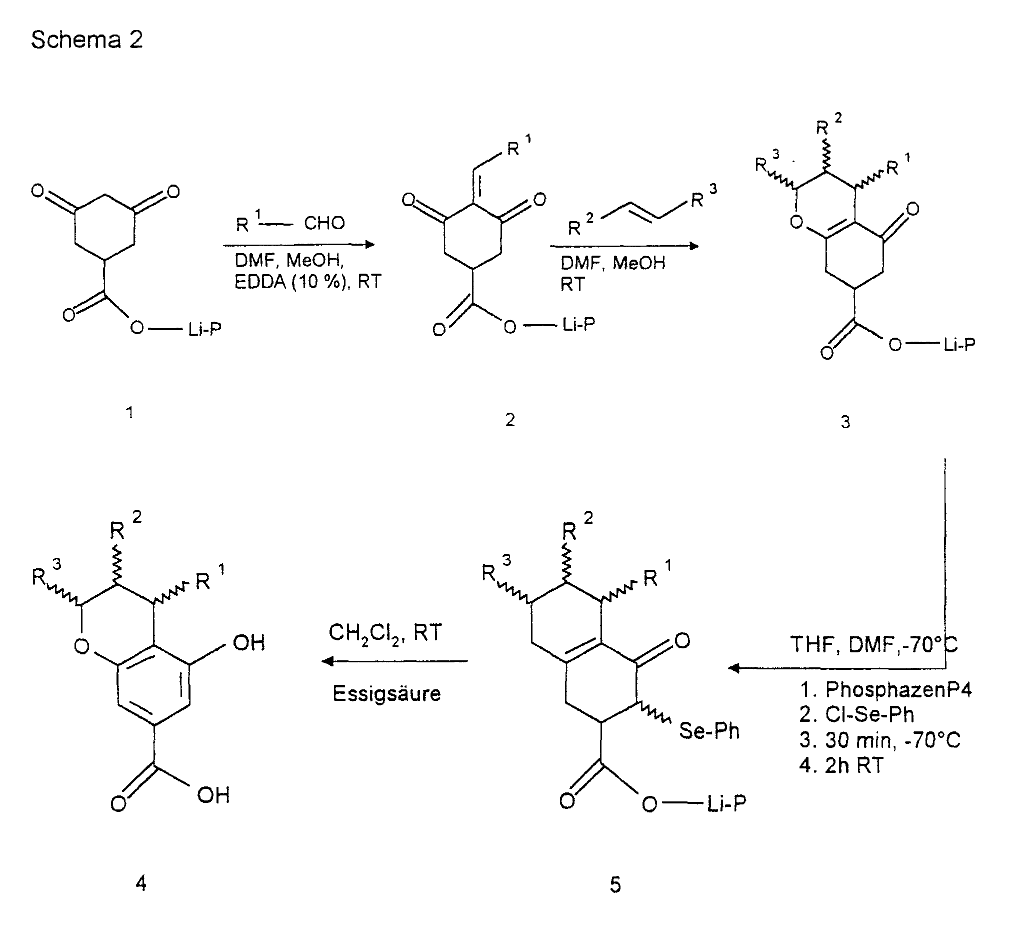

### 1. Synthese von (3)

300 mg (1) werden in 0.2 ml Methylenchlorid vorgequollen und mit 60 ul 1-Heptanal sowie 2 ml Methanol versetzt. Man addiert 10 mg Ethylendiammoniumdiaacetat und läßt 2 h bei Raumtemperatur reagieren. Danach werden 0.5 ml tert. Butylvinylether zugefügt und die Suspension weitere 64 h bei Raumtemperatur geschüttelt. Nach Entfernen der Lösung über eine Fritte wird das Harz gründlich mit Methylenchlorid gewaschen. Nach Trocknen verbleiben ca. 330 mg (3). Nach Abspaltung des Polymers durch Versetzen mit Essigsäure/Methylenchlorid und Abdampfen des Lösungsmittels werden 3,4 mg des freien Dyhydropyrans (3, Li-P = H) als Diastereomerenmischung erhalten (HPLC/MS).

### 2. Synthese von (4)

1 g (3) werden in 6 ml DMF abs. und 4 ml THF abs. unter Argon auf -70°C gekühlt. Nach Zugabe von 2,5 ml PhosphazenP₄ läßt man 3h bei -70°C reagieren. Man gibt dann eine Lösung von 191 mg Phenylselenylchlorid in 2ml THF abs. zu, und lässt nach 30 min bei -70°C und 2h bei Raumtemperatur reagieren. Danach wird mit DMF abgesaugt und mit Puffer, pH6, CH₃OH und MTB + Toluol gewaschen. Das Harz wird über Nacht mit 9:1 CH₂Cl₂/Essigsäure bei Raumtemperatur abgespalten. Man erhält 118 mg (4).

### C. Synthese von 1,3-Dioxan annelierten Dihydropyranen (Schema 3)

hier beispielhaft mit R¹ = Hexyl

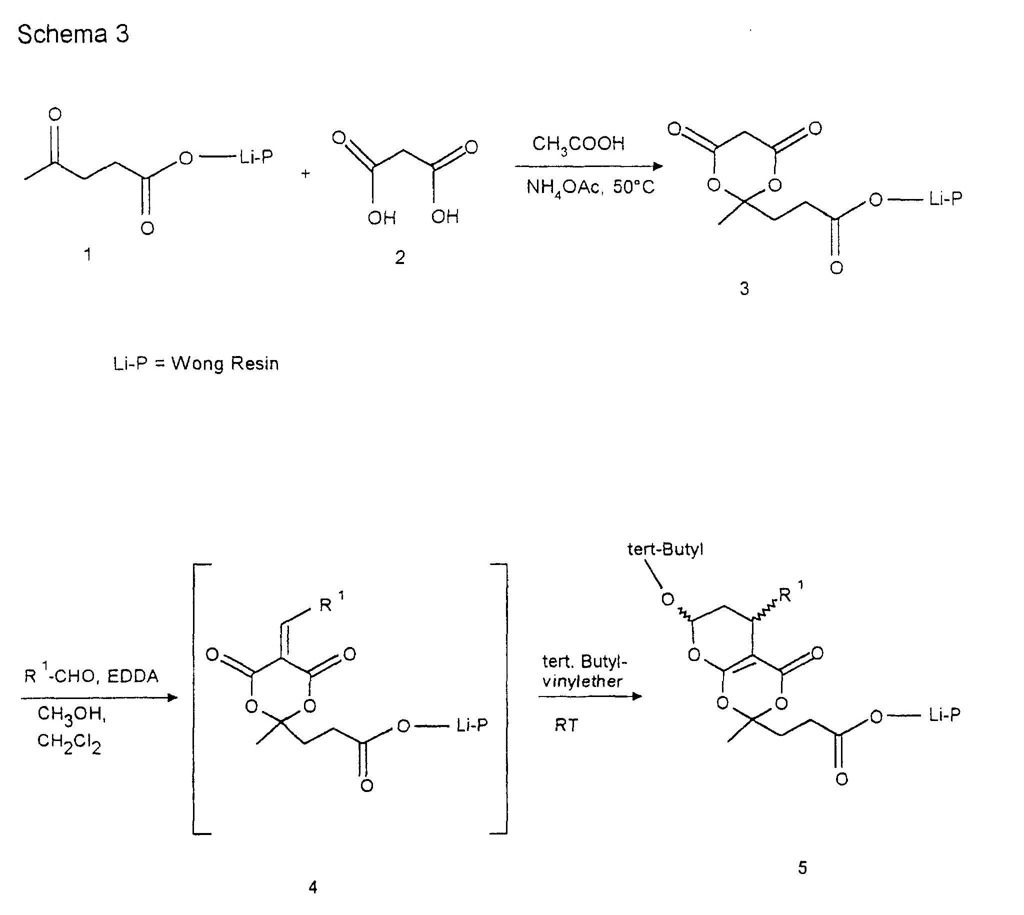

### 1. Synthese des Harz gebundenen Meldrumsäure-Derivats (3)

50 g Harz gebundene 2-Oxo-pentancarbonsäure (1) wird in 200 ml Essigsäure gequollen und mit 20 g Malonsäure (2) versetzt. Man addiert 10 g wasserfreies Ammoniumacetat und erhitzt auf 50 oC. Nach 3 h vorsichtigem Rühren wird das Harz von der Lösung befreit, gründlich mit DMF und Methylenchlorid gewaschen und getrocknet. Man erhält 53 g (3). Eine Probeabspaltung mit wasserfreier TFA liefert das freie Meldrumsäure-Derivat (3, Li-P = H) (HPLC,MS).

### 2. Synthese von (5)

1g (3), EDDA, 3ml CH₂Cl₂, 20 ml CH₃OH abs., 300 µl n-Heptanal läßt man 2 min bei Raumtemperaturen reagieren. Nach Zugabe von 3ml tert.-Butylvinylether läßt man 24 h bei Raumtemperatur reagieren. Das Harz wird abgesaugt und mit DMF und MTB-Ether gewaschen (5). Das Harz wird in 9:1 CH₂Cl₂/Essigsäure über 24h bei Raumtemperatur abgespalten, filtiriert und eingedampft.

### D. 1,3 -Pyrimidin-2-on annelierte Dihydropyrane können beispielsweise nach folgenden Schema synthetisiert werden (Schema 4)

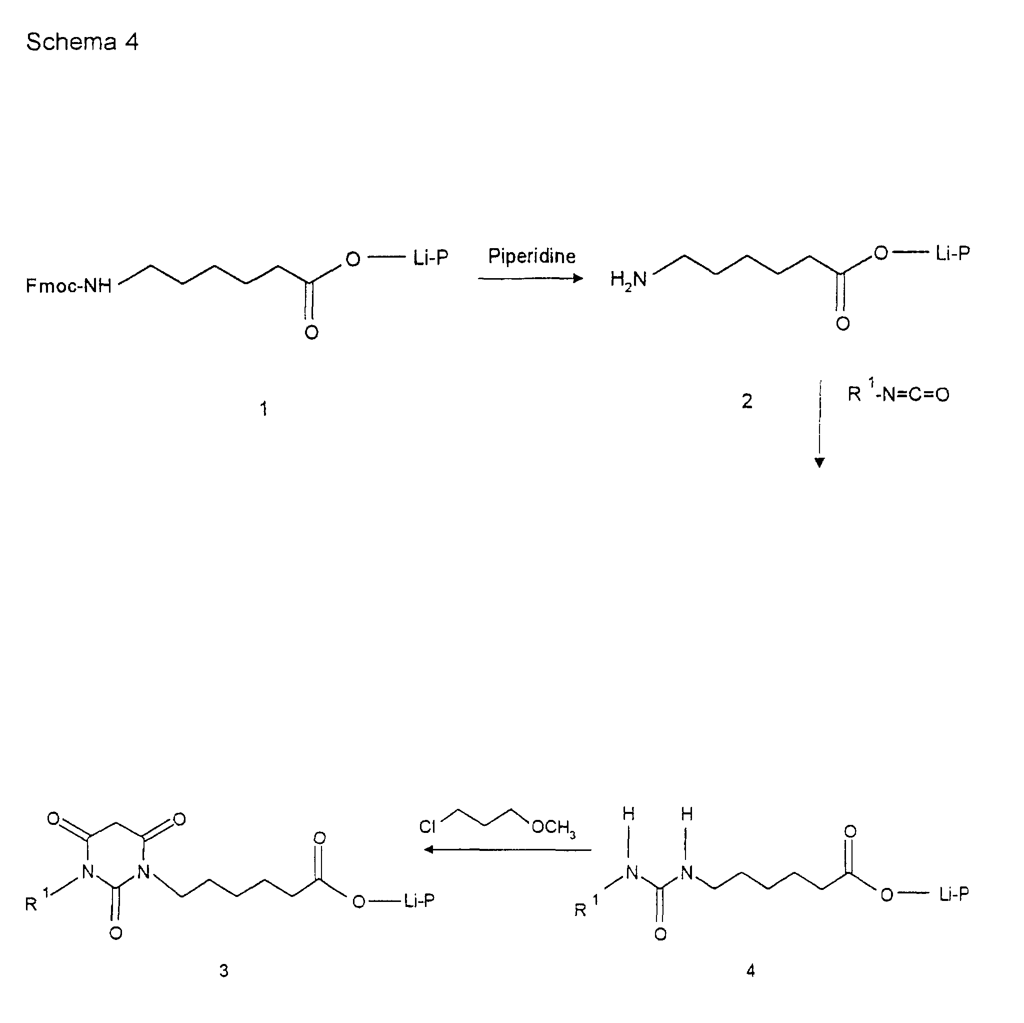

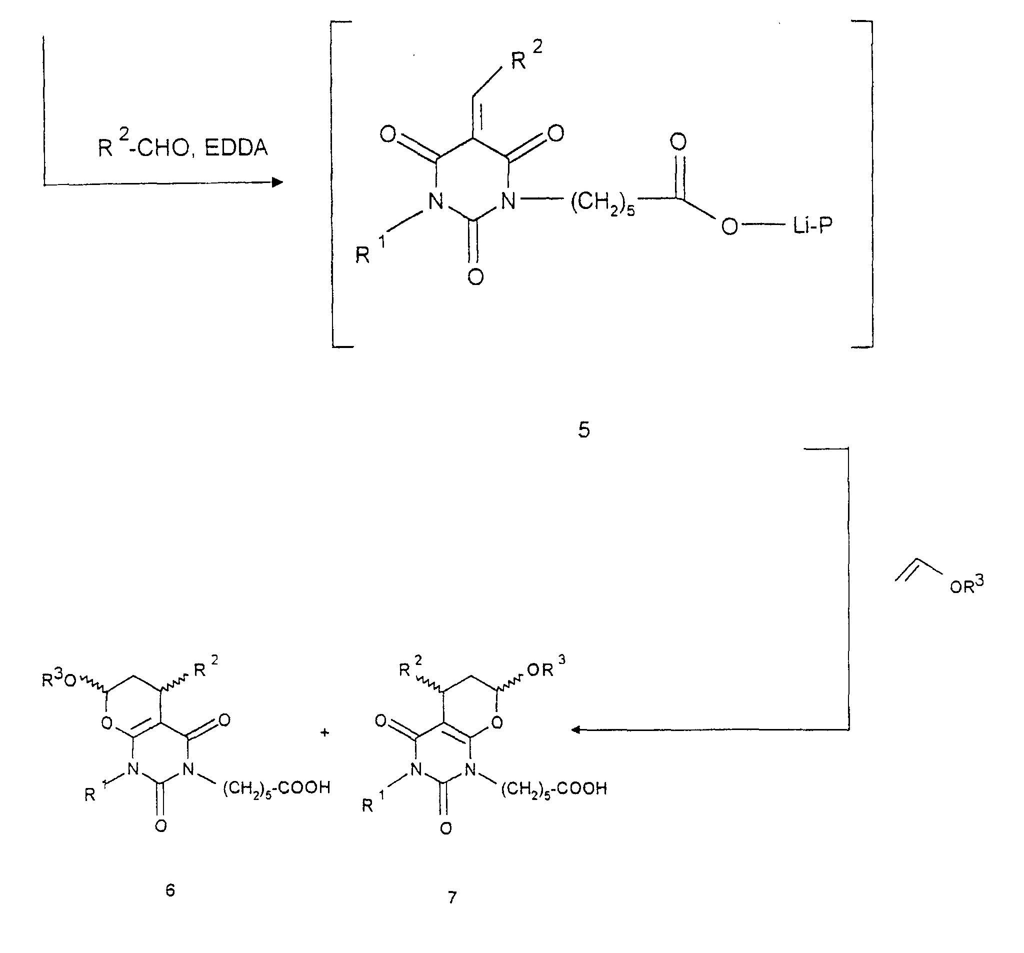

### 1. Synthese von (4)

Ca. 200 mg Polymer- gebundene geschützten Aminohexancarbonsäure (Fa. Novabiochem) wird in Lösungsmittel (z.B. DMF) gequollen und mit ca. 0.5 ml Piperidin versetzt. Man rührt ca. 30 min bei Raumtemperatur, befreit das Harz von der Lösung und wäscht gründlich mit Lösungsmittel (z.B. DMF). Man nimmt erneut in Lösungsmittel auf (z.B. 2 ml DMF) und addiert ca. 1 mmol des Isocyanats sowie ca. 0.3 ml Triethylamin. Man rührt ca. 8 h bei Raumtemperatur. Anschließend wird das Harz von der Lösung befreit und gründlich mit Lösungsmittel (z.B. DMF und Methylenchlorid gewaschen. Nach Trocknen erhält man (4). Das freie Harnstoff-Derivat (4, Li-P = H) wird durch Abspaltung des Linkers erhalten.

### 2. Synthese von (3)

Ca. 200 mg von (4) wird bei Kälte in Lösungsmittel gequollen (z.B. bei 0°C in DMF) und mit ca. 1 mmol Chlormalonsäure-Monomethylester versetzt. Man addiert ca. 0.5 ml Triethylamin und läßt ca. 2 h bei 0°C sowie 1 h bei geringer Hitze (z. B. 50°C) rühren. Nach Waschen des Harzes isoliert man (3). Das freie Derivat der N,N'-Dialkyl-barbitursäure(3, Li-P = H) wird dann durch Abspaltung des Linkers erhalten.

### 3. Synthese von (6) und (7)

Ca. 300 mg von (3) werden mit ca. 0.5 mmol des Aldehyds versetzt und in DMF gequollen. Man addiert ca. 10 mg Ethylendiammoniumdiacetat und ca. 1 ml Methanol sowie 1ml des Enolethers und läßt ca. 8 h bei geringer Hitze (z.B. 40°C) rühren. Nach gründlichem Waschen des Harzes mit Lösungsmittel (z. B. DMF und Methylenchlorid) wird getrocknet. Die Abspaltung vom Trityl-Linker erfolgt mit Essigsäure und Methylenchlorid. Nach Einengen des Lösungsmittels im Vakuum erhält man die Regio- und Diastereomeren (6) und (7).

Ein weiterer Gegenstand der Erfindung sind Arzneimittel, die ein oder mehrere erfindungsgemäße Verbindungen der Formel 1 und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen ( = Wirkstoff) entweder als solche, oder vorzugsweise in Kombination mit geegneten pharmazeutischen Hilfsstoffen in Form von Tabletten, Dragees, Kapseln, Suppositorien, Emulsionen, Suspensionen, Granulaten, Pulvern, Lösungen oder Präparate mit protalierter Wirkstoff-Freigabe, eingesetzt, wobei der Wirkstoffgehalt vorteilhafterweise 0,1 bis 95 % beträgt.

Welche Hilfstoffe für die gewünschte Arzneimittelformulierung geeignet sind, ist dem Fachmann aufgrund seines Fachwissens geläufig. Neben Lösungsmitteln, Gelbildnern, Suppositoriengrundlagen, Tabletten-Hilfsstoffen und anderen Wirkstoffträgern können beispielsweise Antioxidatien, Dispergiermittel, Emulgatoren, Entschäumer, Geschmackskorrigentien, Konservierungsmittel, Lösungsvermittler oder Farbstoffe verwendet werden.

Die Wirkstoffe können topisch, oral, parenteral oder intravenös appliziert werden, wobei die bevorzugte Applikationsart von der zu behandelnden Krankheit abhängig ist. Die orale Verabreichung ist bevorzugt.

Für eine orale Anwendungsform werden die aktiven Verbindungen mit den dafür geeigneten Zusatzstoffen wie Trägerstoffen, Stabilisatoren oder inerten Verdünndungsmittel vermischt und durch üblichen Methoden in geeigneten Dareichungsformen gebracht, wie Tabletten, Dragees, Steckkapseln, wässrige, alkoholische oder ölige Suspensionen oder wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen pflanzliche oder tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus verschiedenen Lösungsmitteln.

Als pharmazeutische Präparate für topische und lokale Verwendung eignen sich Augentropfen, welche die aktive Verbindung in wässriger oder öliger Lösung enthalten. Für die Anwendung an der Nase sind Aerosole und Sprays, sowie grobe Puder, die durch schnelles Inhalieren durch die Nasenlöcher verabreicht werden, und vor allem Nasentropfen, welche die aktive Verbindungen in wässriger oder öliger Lösung enthalten, geeignet.

Die Dosierung des zu verabreichenden Wirkstoffs der Formel I und die Häufigkeit der Verabreichung hängen von der Wirkstärke und Wirkdauer der verwendeten erfindungsgemäßen Verbindung ab; außerdem auch von der Art und Stärke der zu behandelnden Krankheit sowie von Geschlecht, Alter, Gewicht und individuellen Ansprechbarkeit des zu behandelnden Säugers. Im Durchschnitt liegt die empfohlene tägliche Dosis einer erfindungsgemäßen Verbindung bei einem etwa 75 Kg schweren Säuger - in erster Linier einem Menschen - im Bereich von etwa 10 bis 500 mg, vorzugweise von etwa 25 bis 250 mg, wobei die Verabreichung nach Bedarf in mehreren Dosen pro Tag erfolgen kann.

Die in den Tabellen 1-5 aufgeführten Verbindungen lassen sich nach den oben beschriebenen Verfahren herstellen.

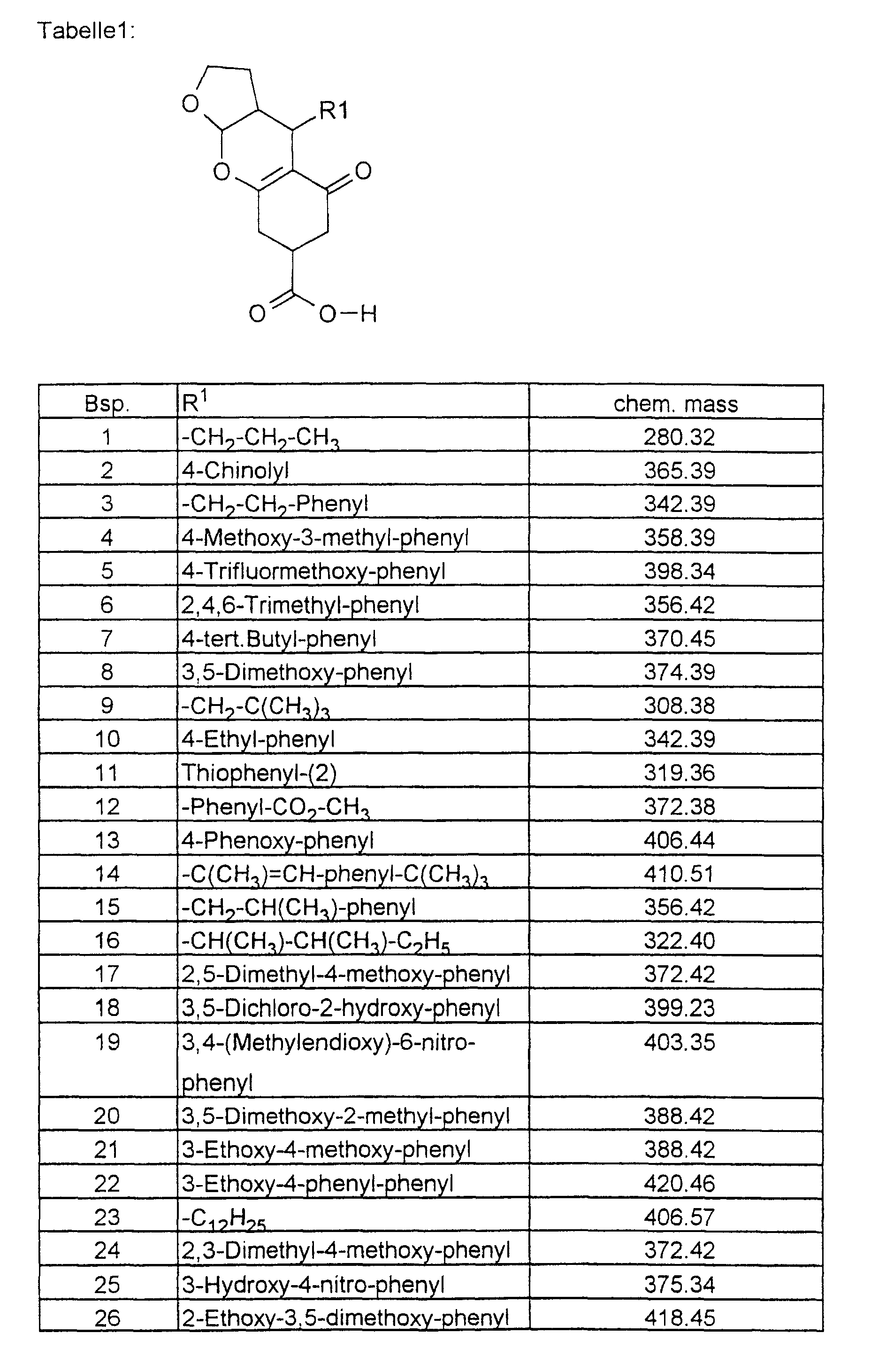

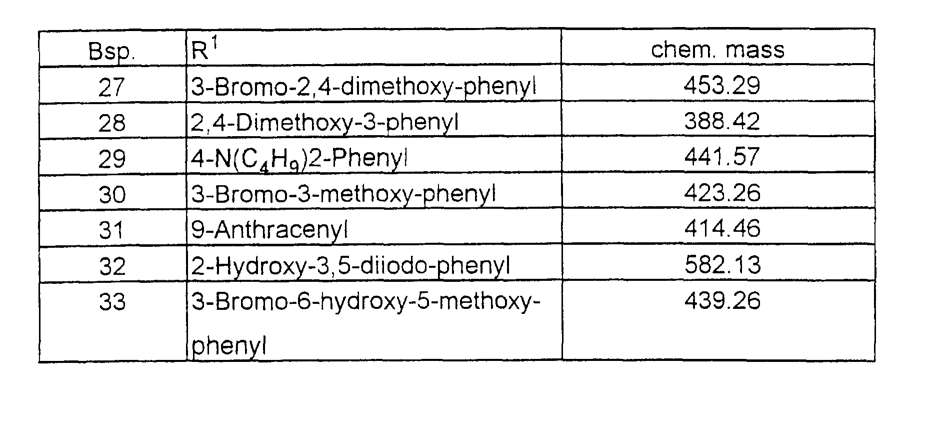

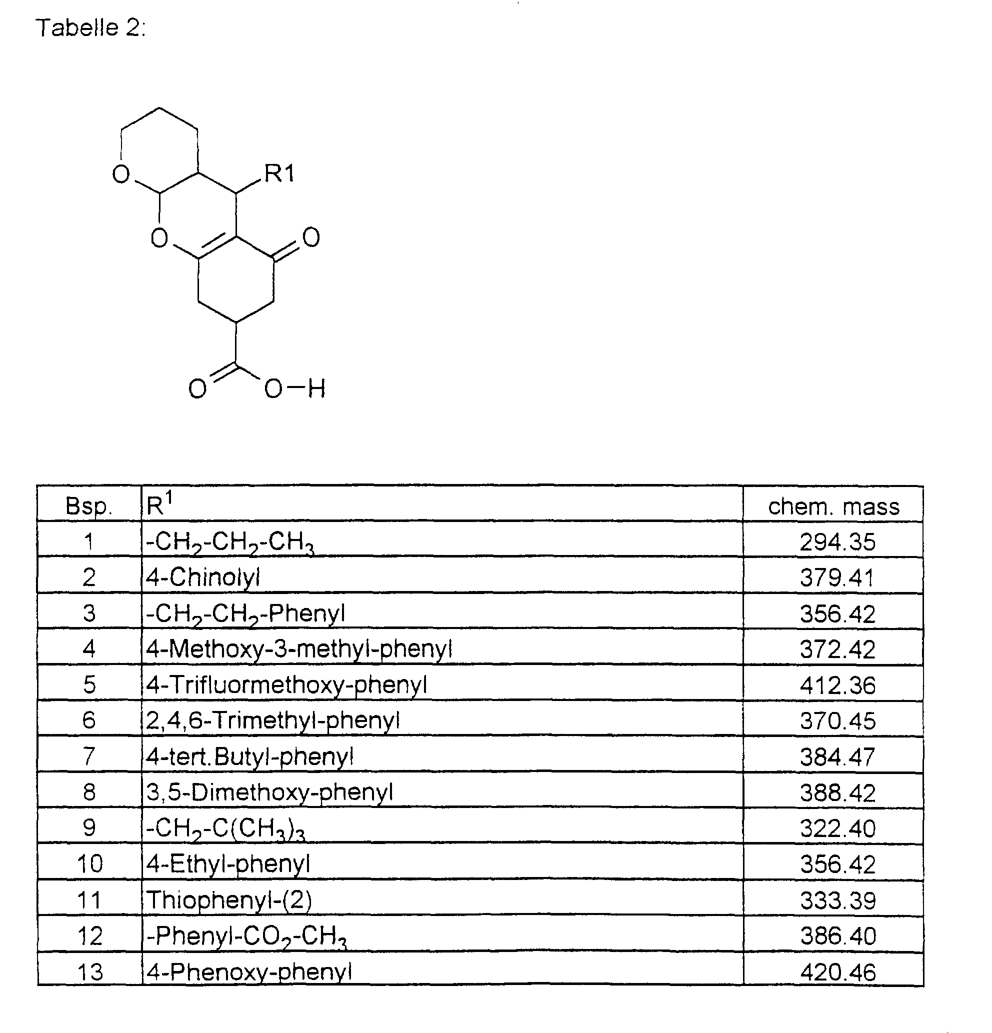

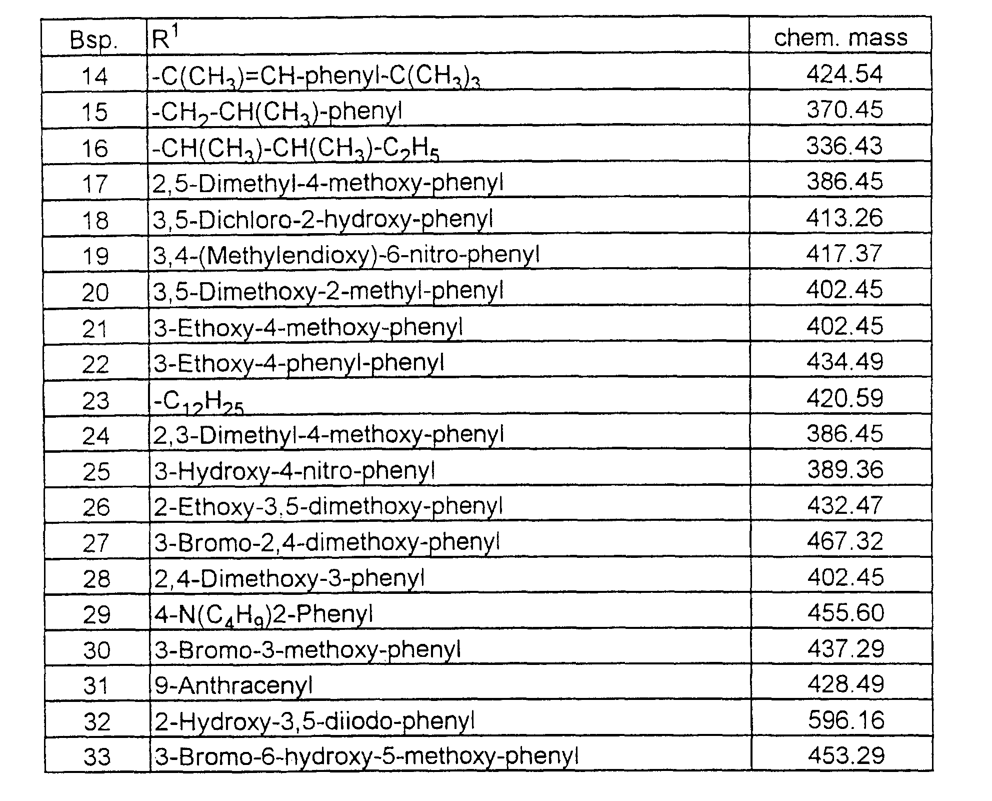

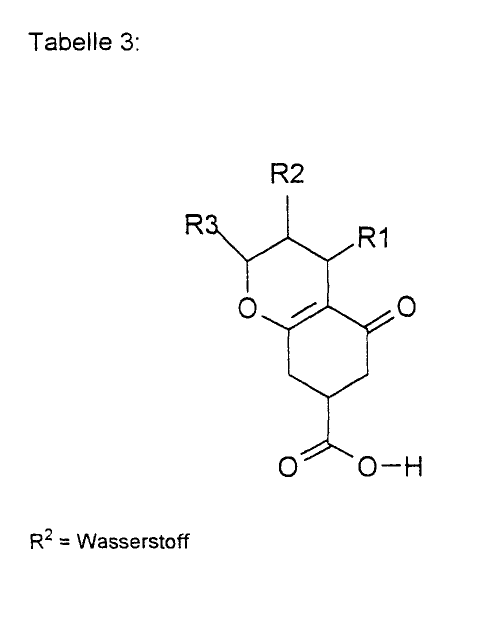

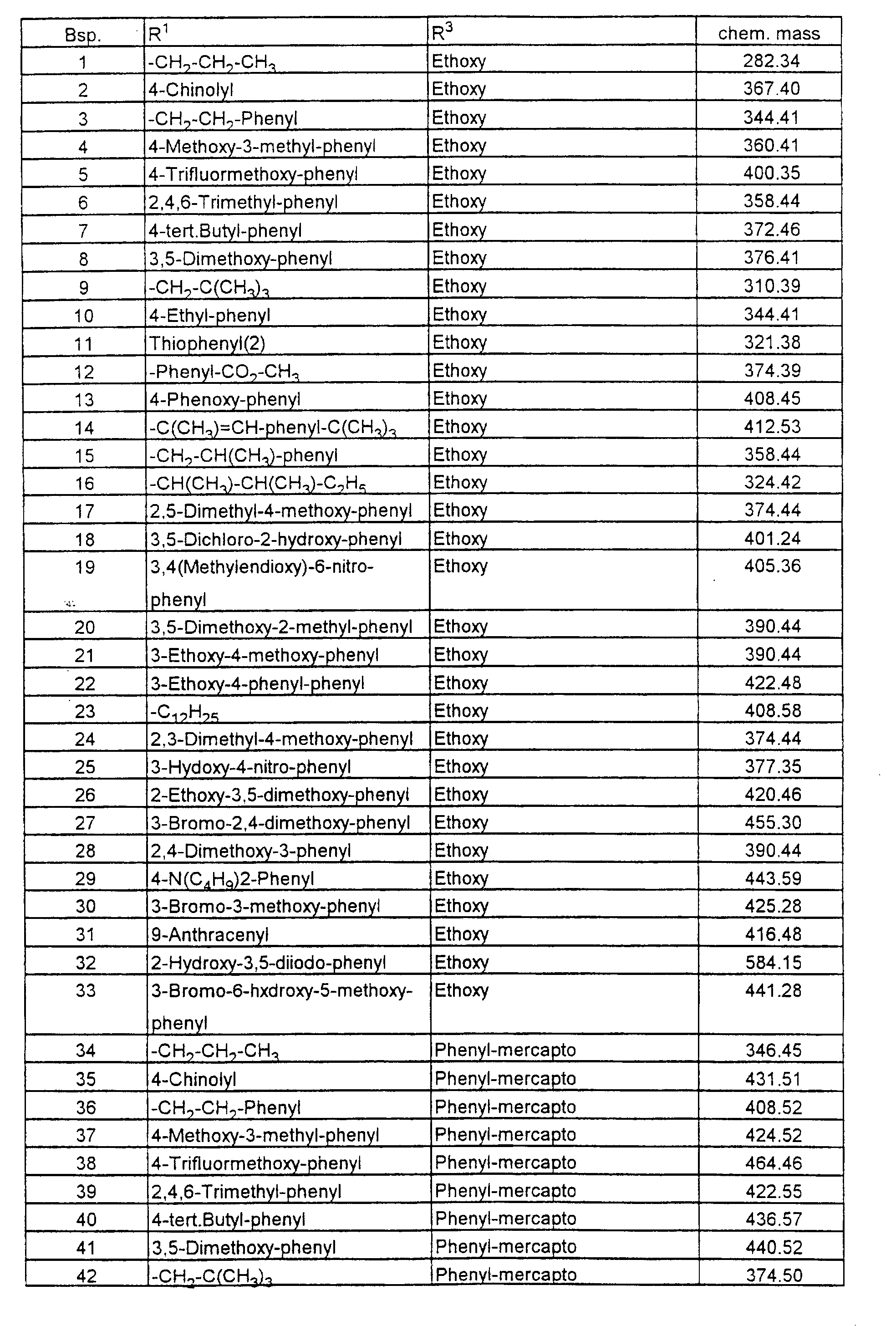

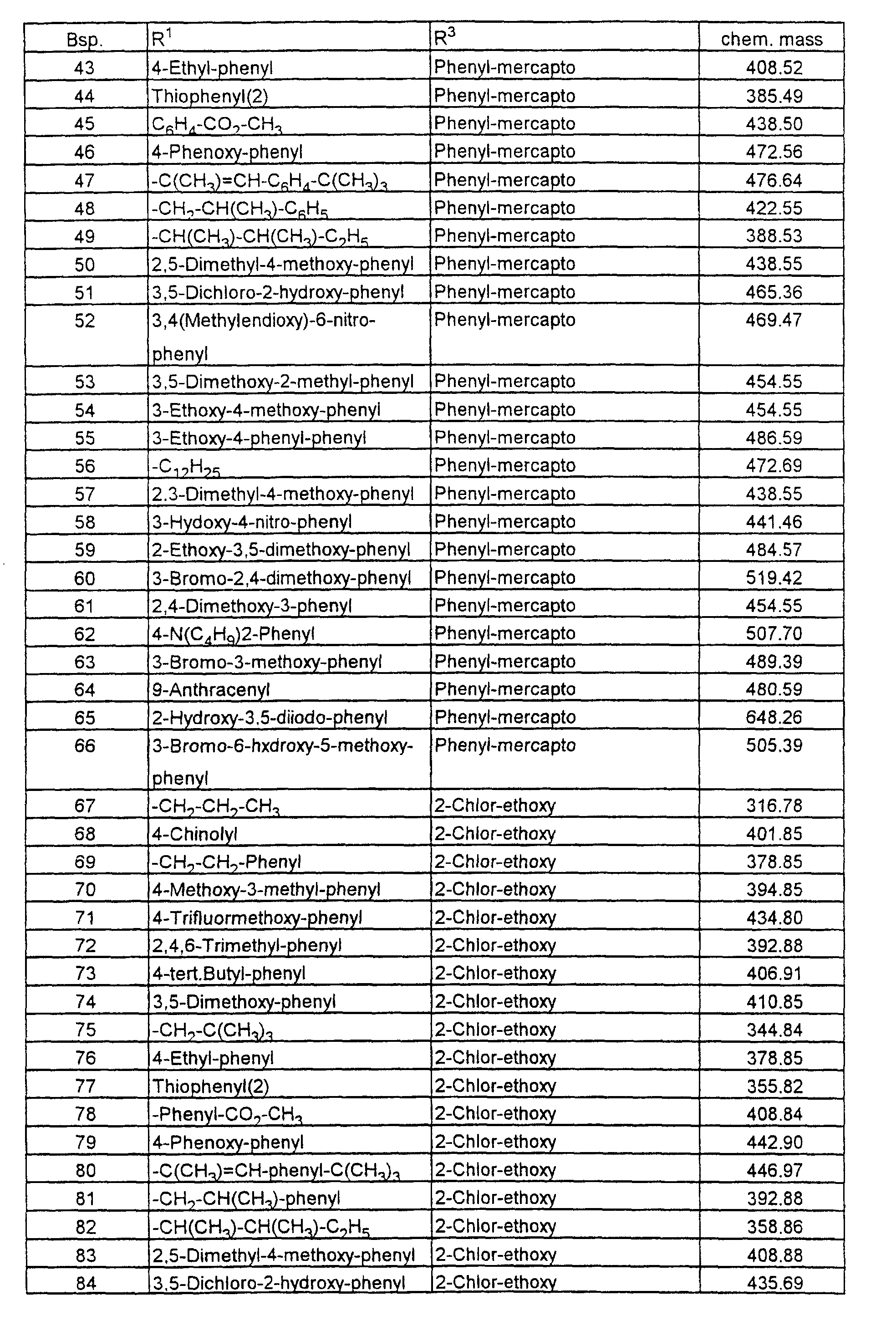

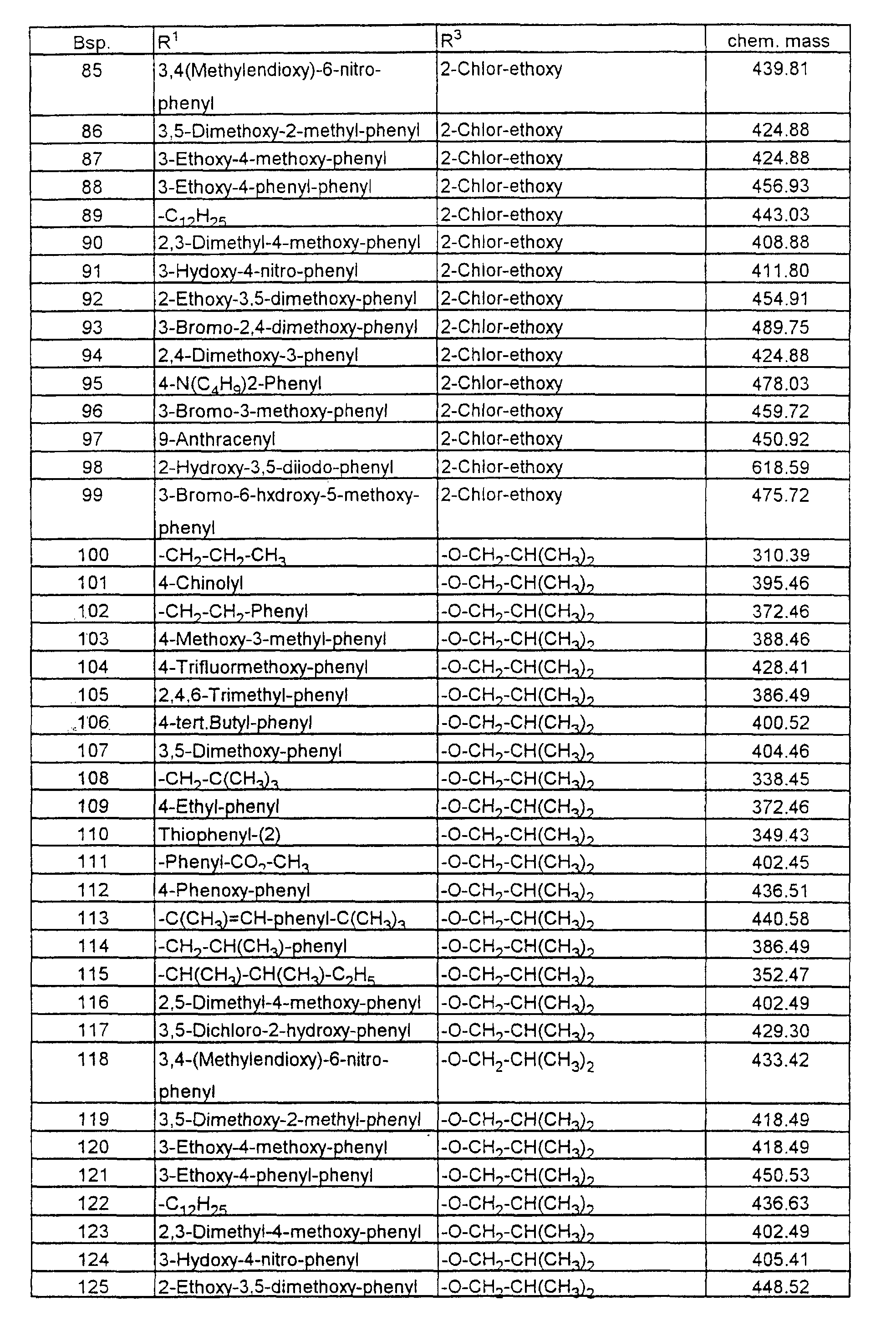

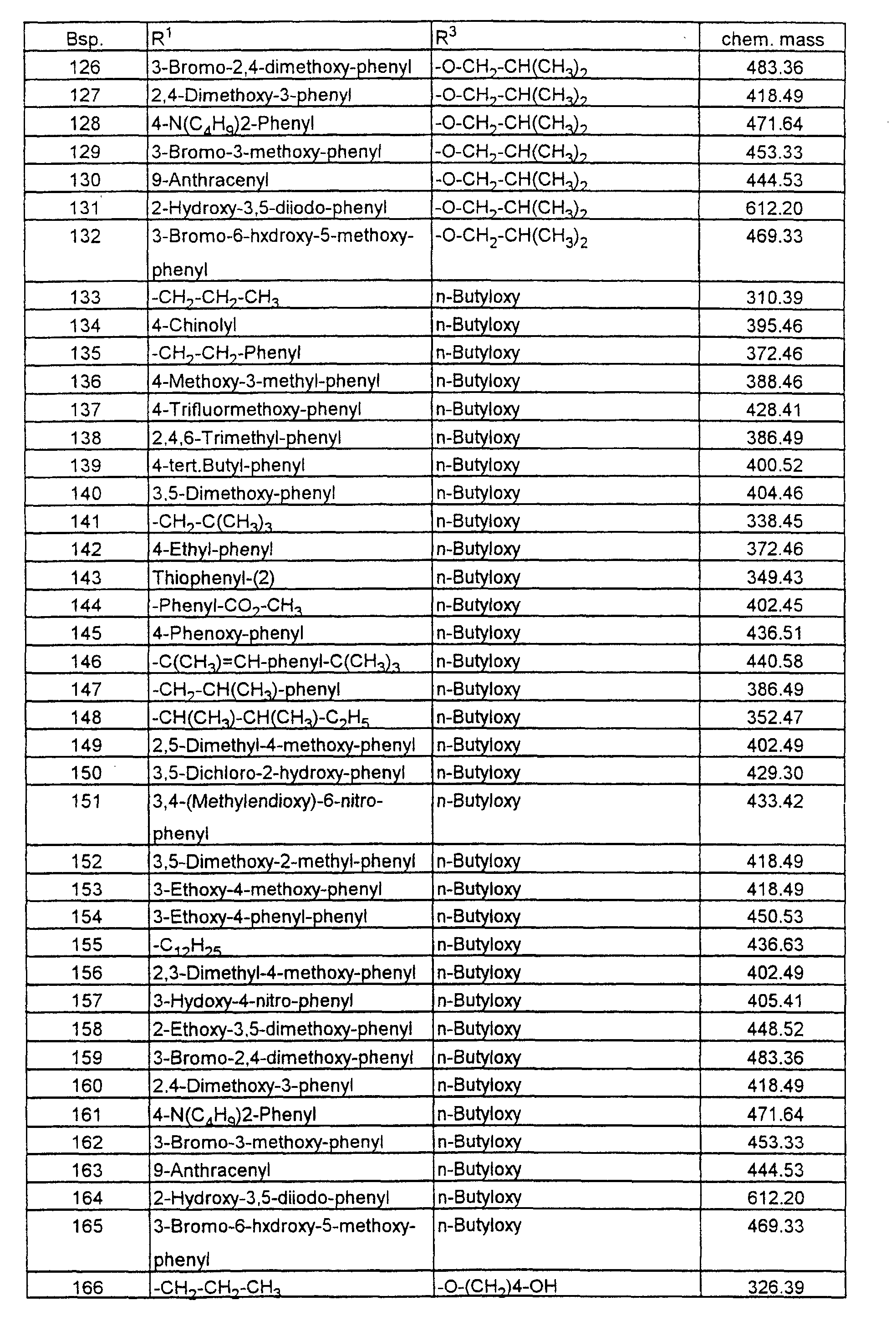

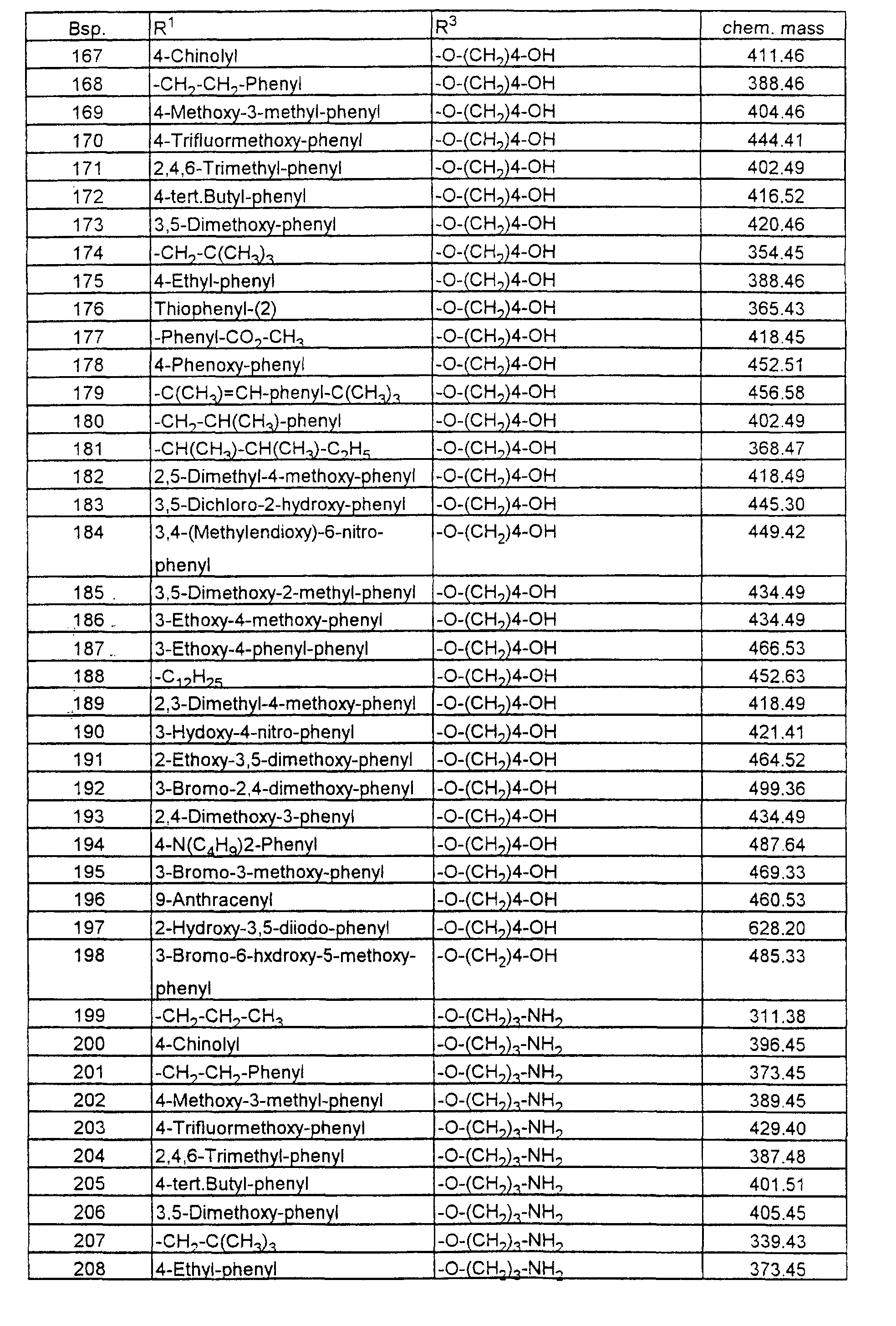

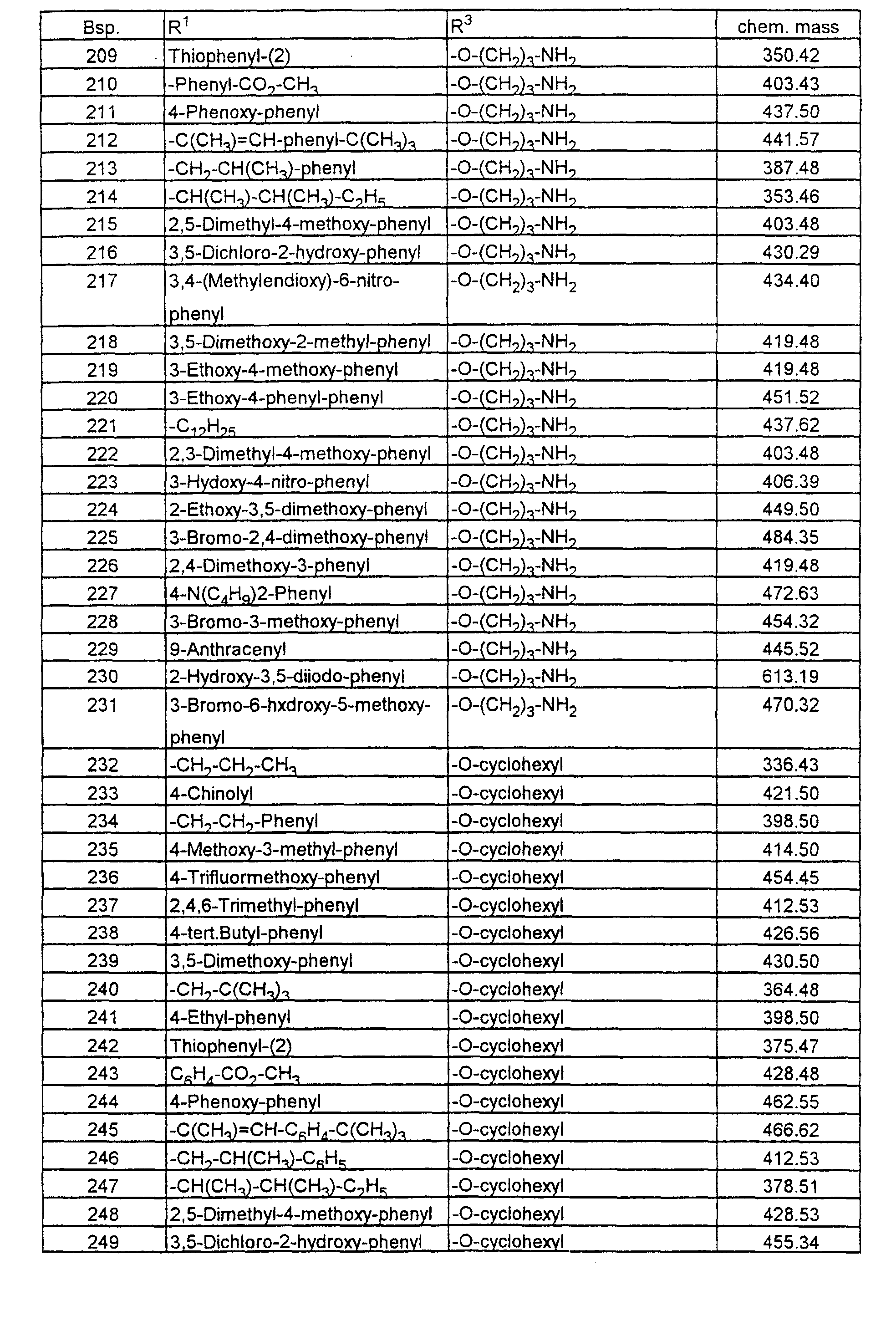

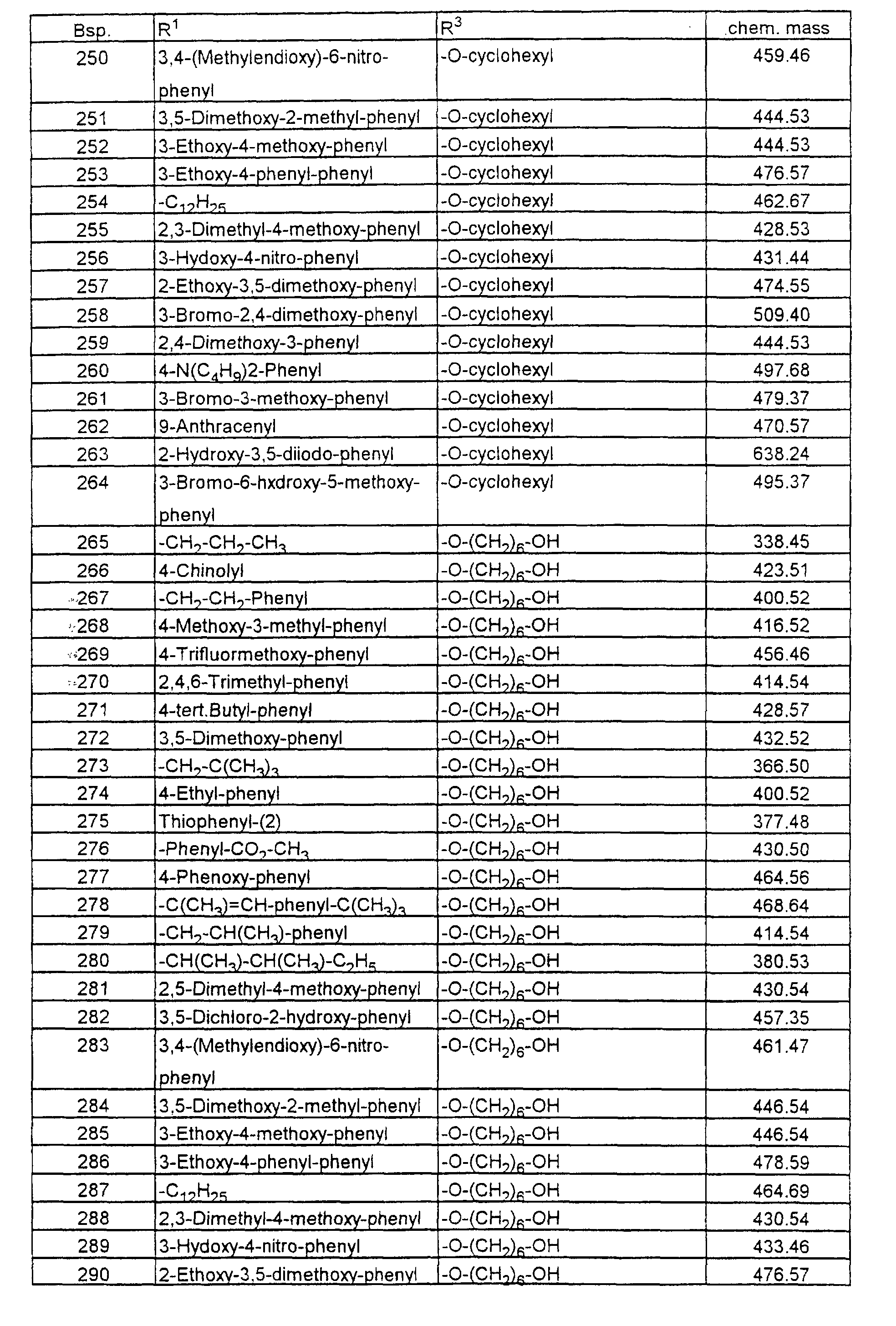

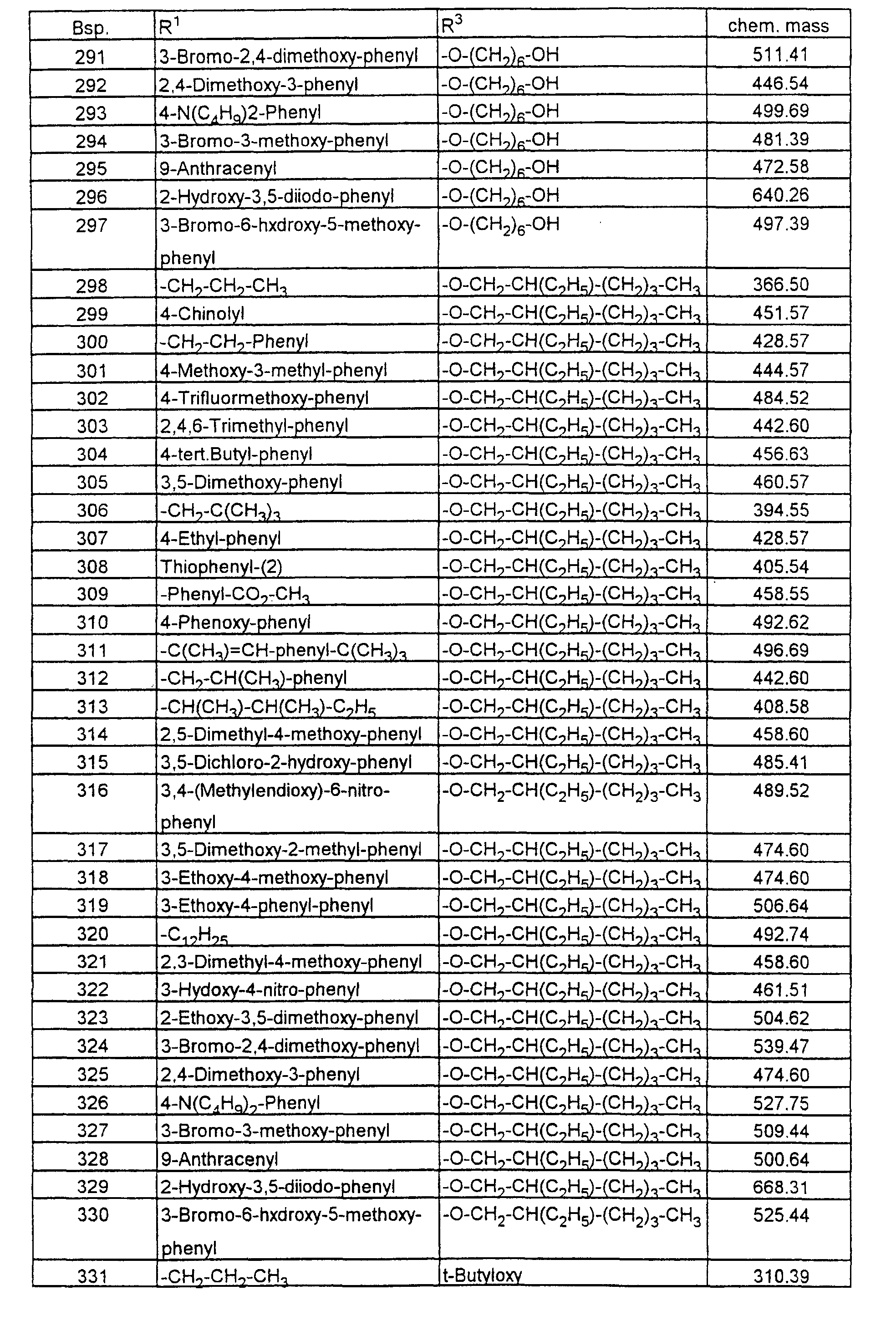

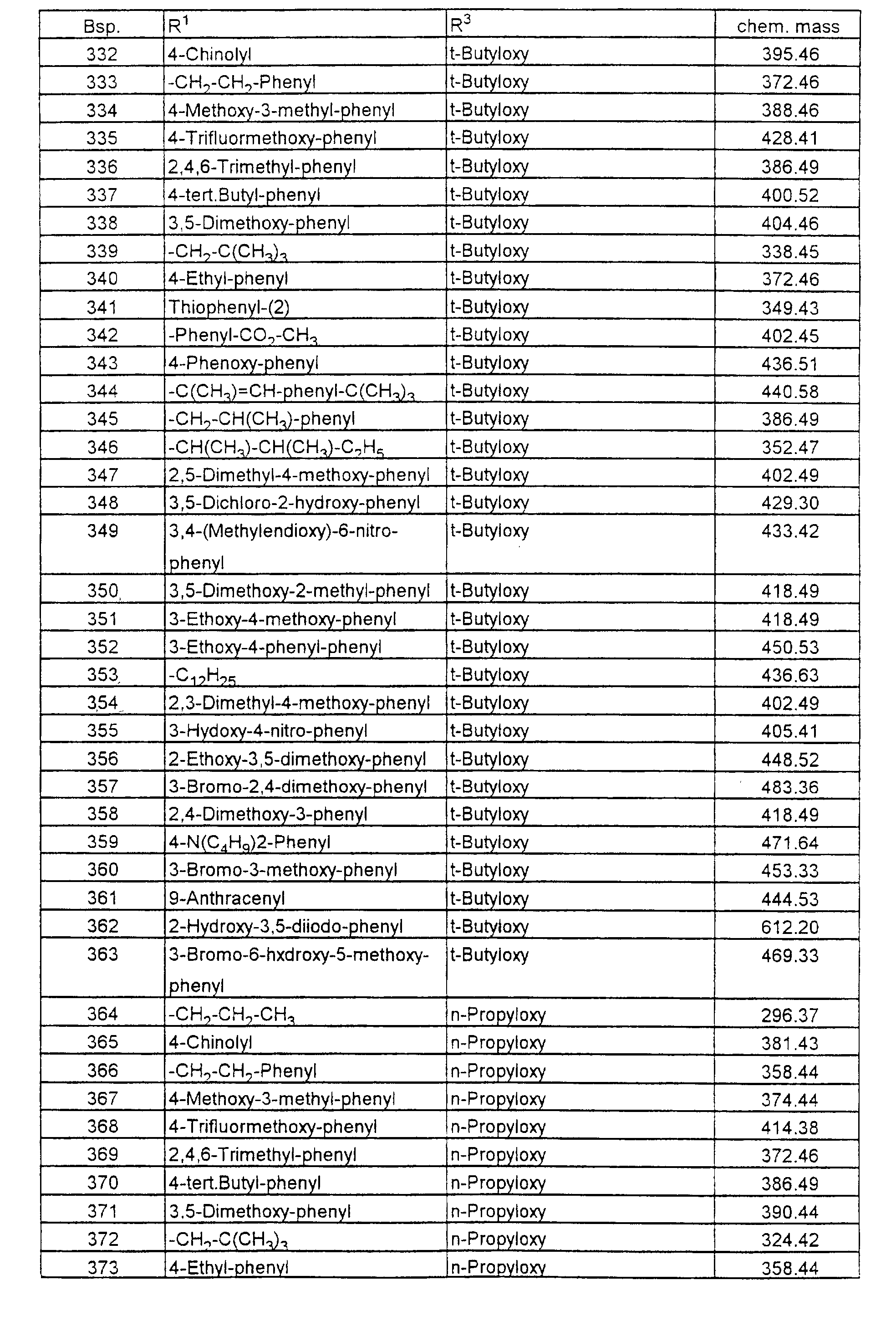

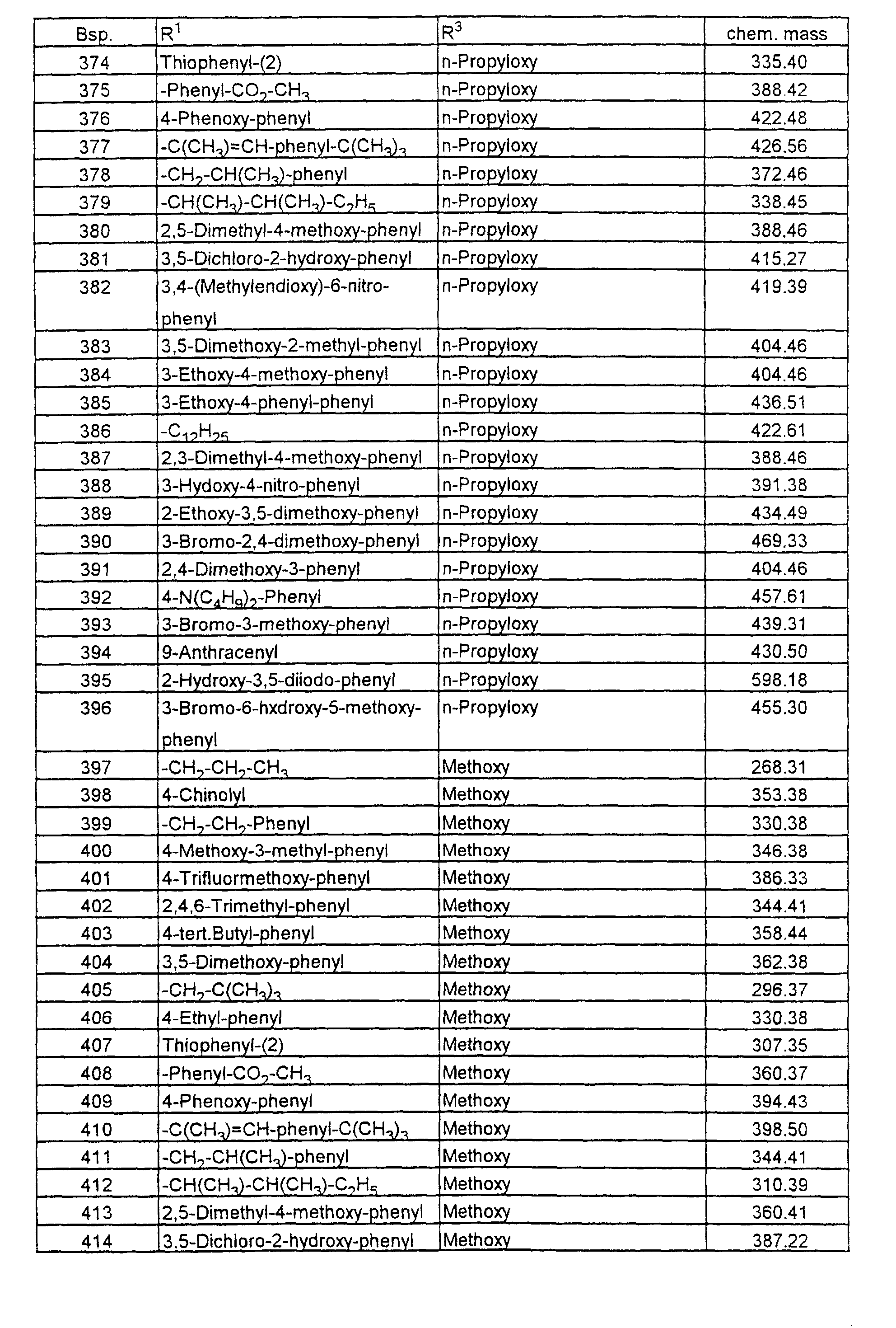

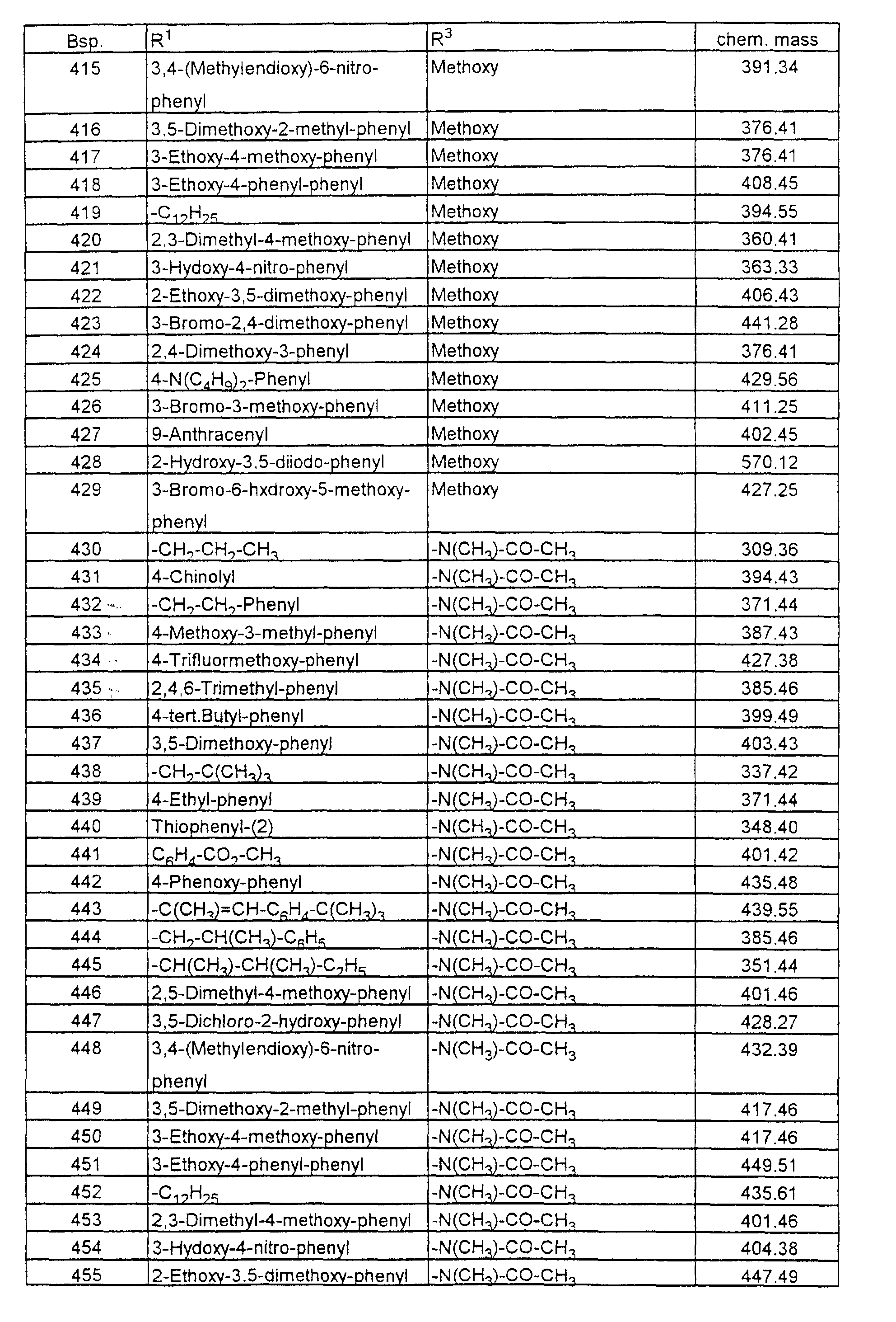

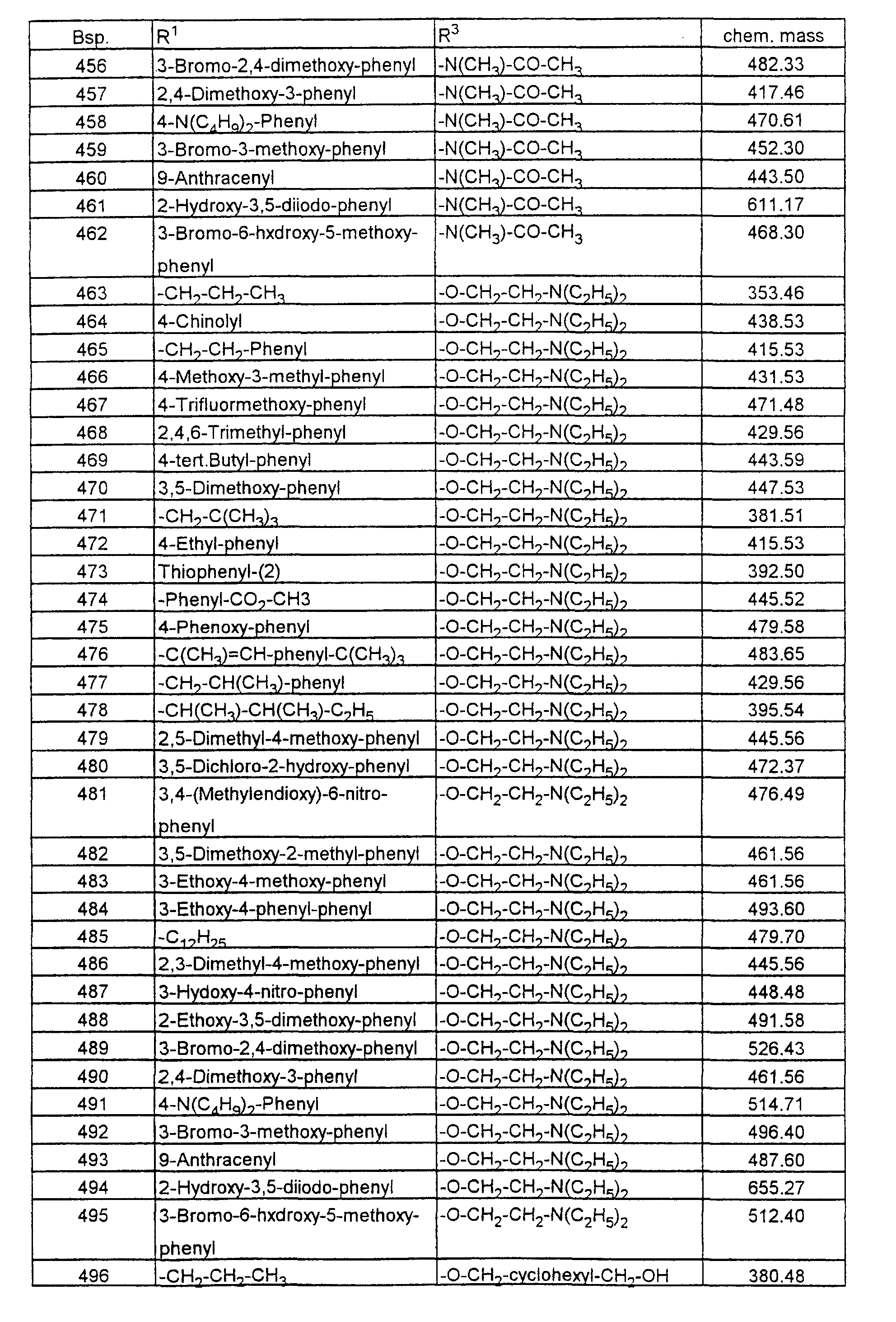

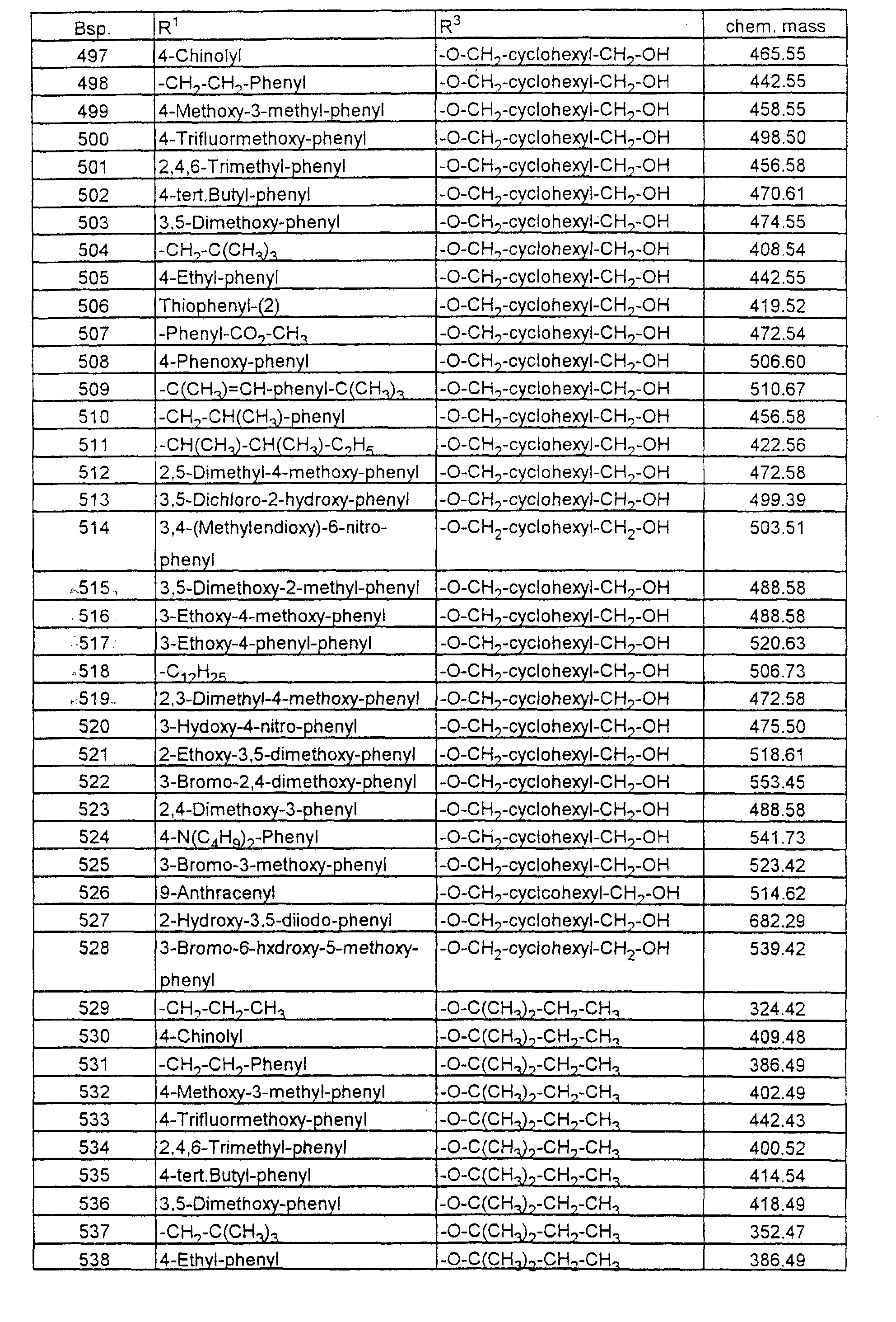

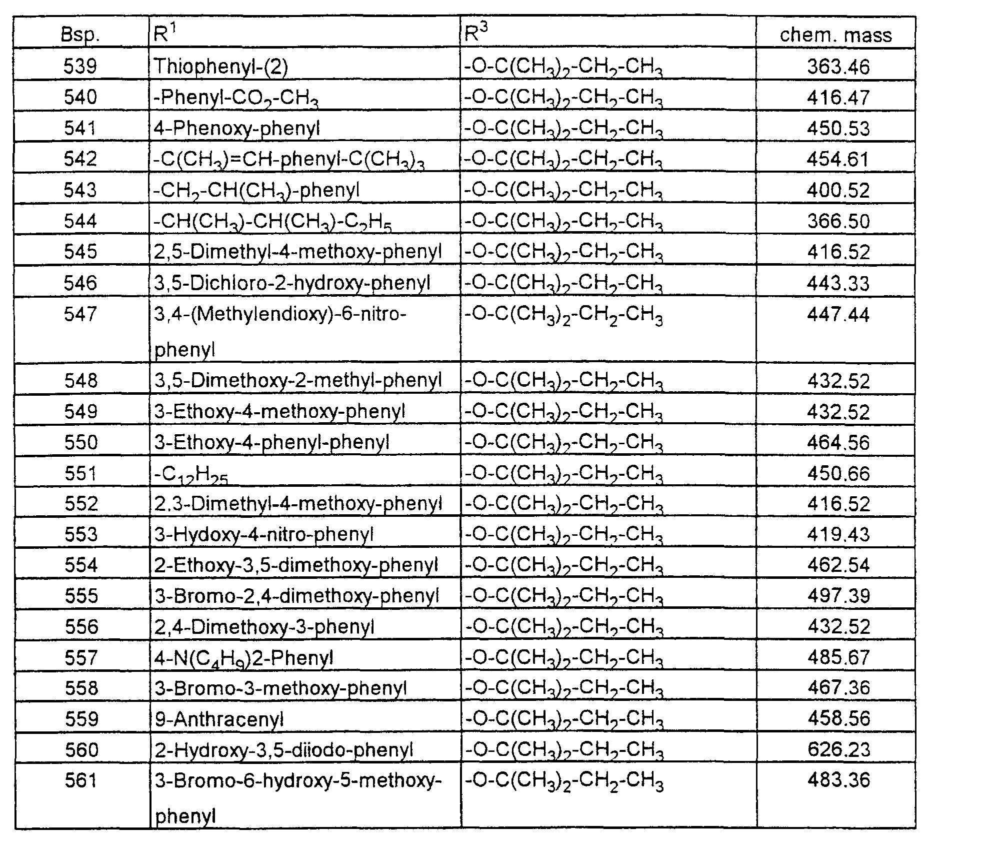

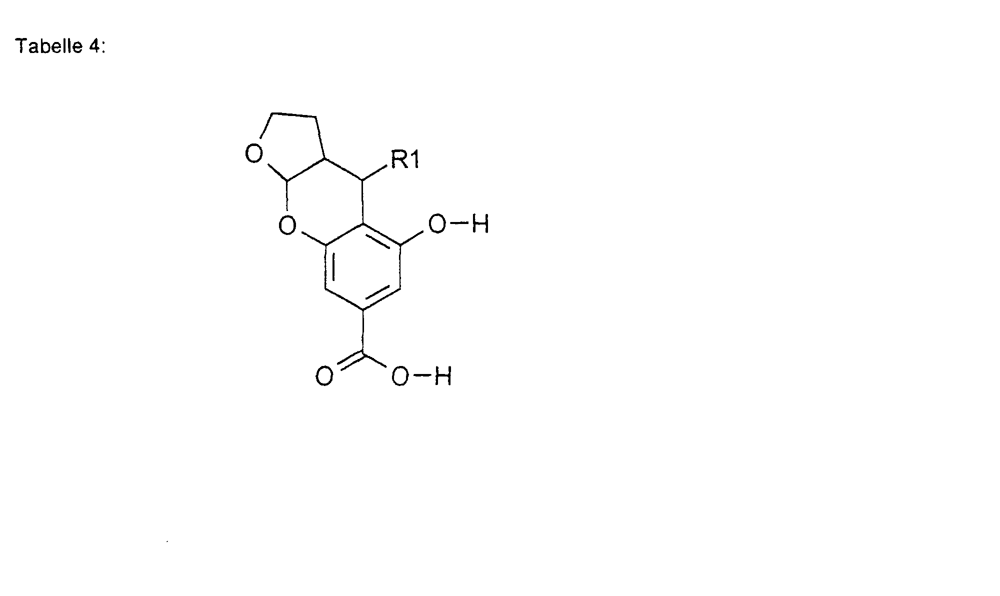

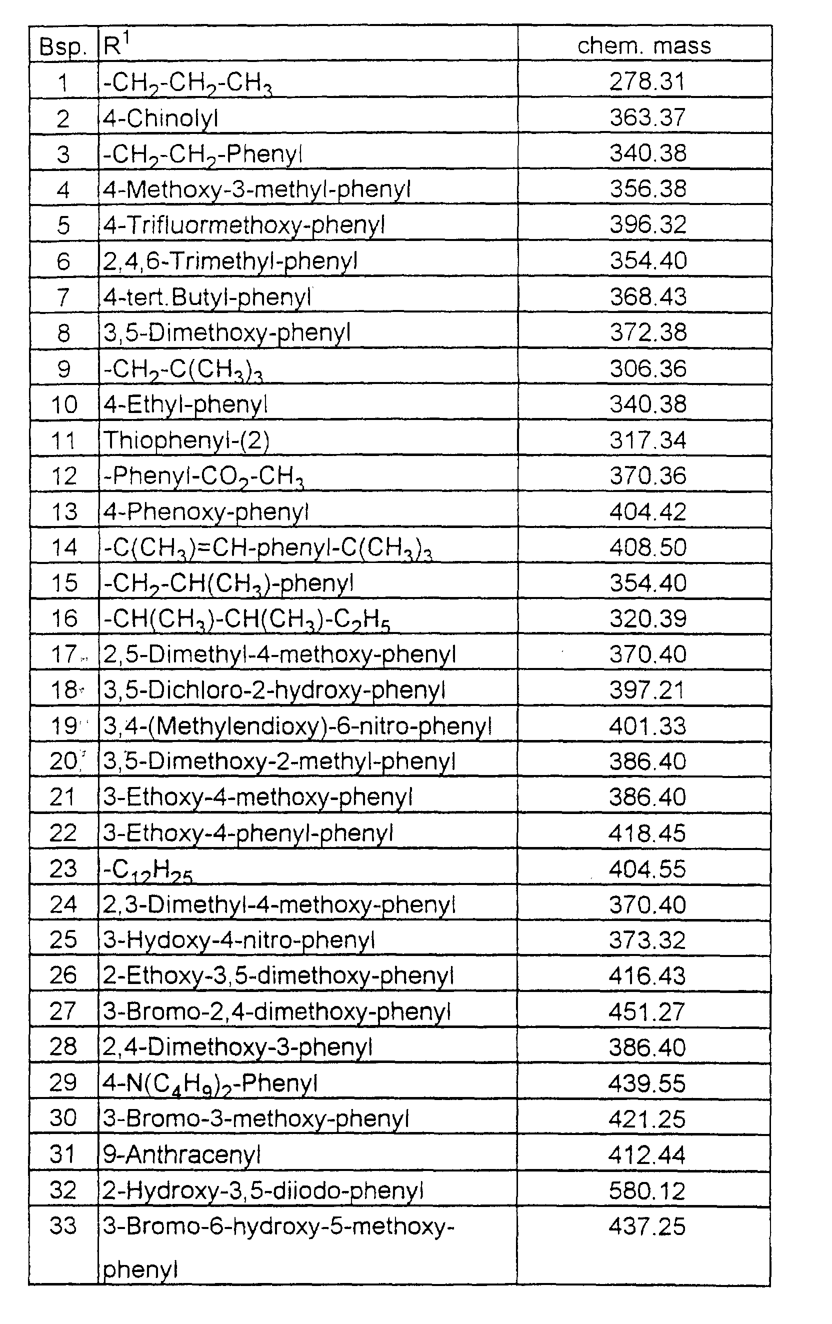

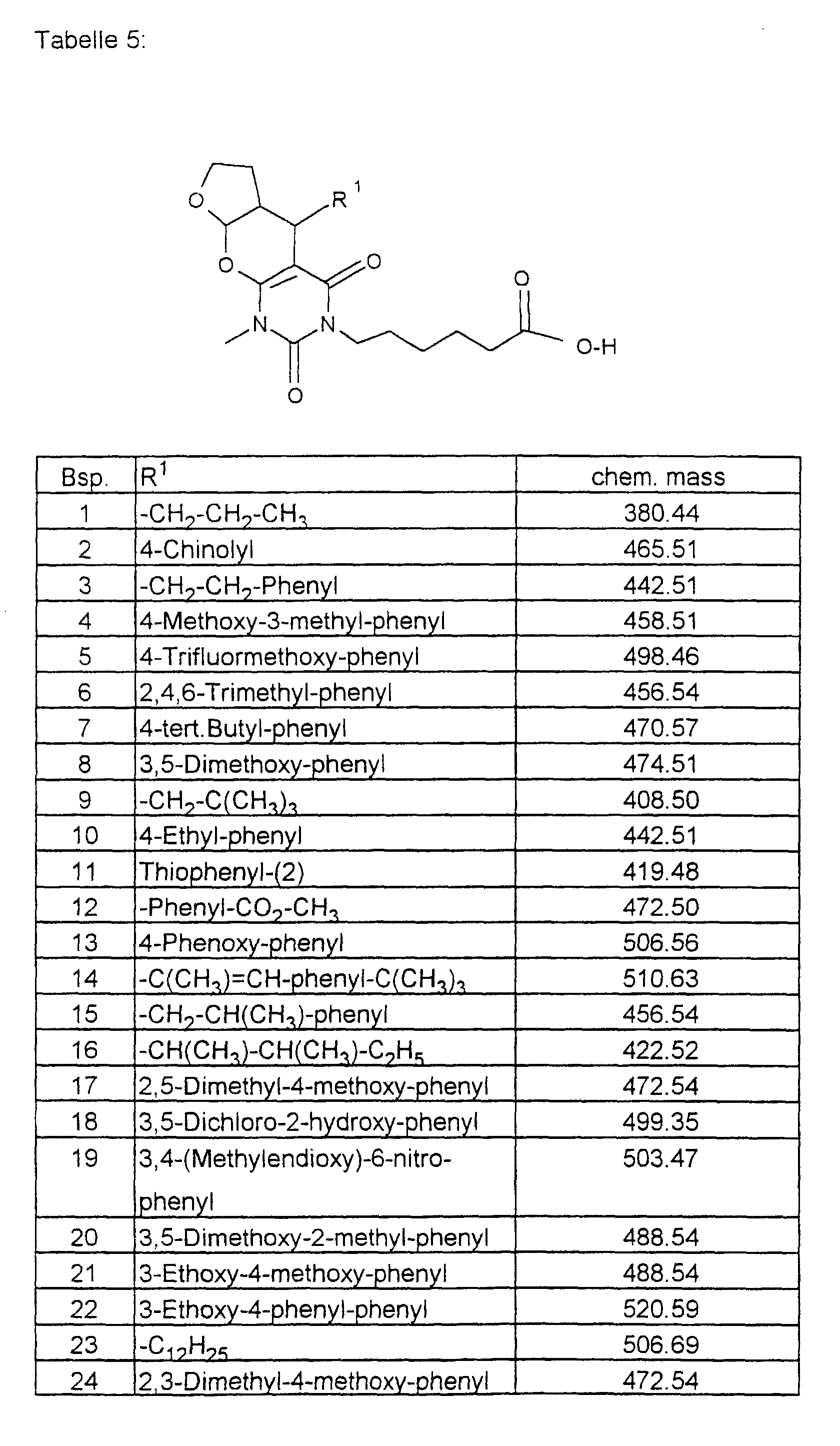

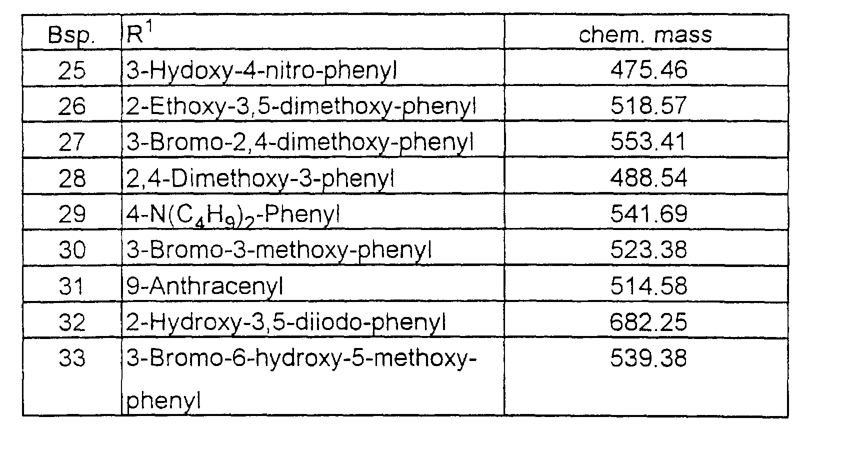

## Patentansprüche

1. Verbindungen der Formel I 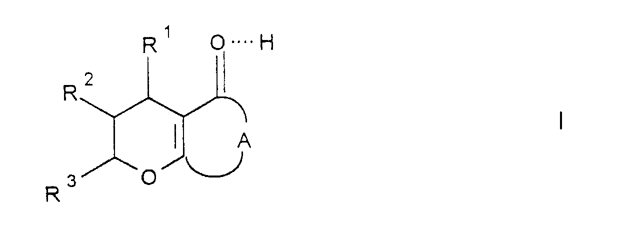 in welcher bedeuten:
R¹
1. (C₁-C₁₄)-Alkyl,
2. (C₂-C₆)-Alkenyl,
3. (C₀-C₆)-Alkyl-(C₃-C₁₀)-cycloalkyl-(C₀-C₆)-alkyl, wobei der Alkylteil gegebenfalls mit einer oder mehreren OH-Gruppen substituiert ist,
4. (C₀-C₆)-Alkyl-(C₆-C₁₄)-Aryl,
5. (C₀-C₆)-Alkyl-(C₃-C₉)-Heteroaryl,
6. (C₂-C₆)-Alkenyl-(C₆-C₁₄)-Aryl,
7. (C₂-C₆)-Alkenyl-(C₃-C₉)-Heteroaryl,
8. (C₁-C₆)-Alkanoyl,
9. einen wie unter 4.,5.,6. oder 7. definierten Rest, wobei der (C₆-C₁₄)-Aryl-, bzw. (C₃-C₉)-Heteroarylteil mit 1, 2 oder 3 gleichen oder verschiedenen Resten aus der Reihe (C₁-C₁₀)-Alkyl, Carboxy, Amino, Nitro, (C₁-C₄)-Alkylamino, Hydroxy, (C₁-C₄)-Alkoxy, wobei ein bis alle Wasserstoff-Atome durch Fluoratome ersetzt sein können, (C₆-C₁₂)-Aryloxy, Halogen, Cyano, Di-(C₁-C₄)-Alkylamino, Carbamoyl, Sulfamoyl und (C₁-C₄)-Alkoxycarbonyl substituiert ist, oder zwei benachbarte Reste am (C₆-C₁₂)-Arylring zusammen Alkylendioxy, vorzugsweise Methylendioxy bedeuten;
R² Wasserstoff, (C₁-C₁₀)-Alkyl, (C₀-C₆)-Alkyl-(C₆-C₁₂)-Aryl (C₀-C₆)-Alkyl-(C₃-C₉)-Heteroaryl, wobei der C₆-C₁₂-Aryl-, bzw. C₃-C₉-Heteroarylteil gegebenenfalls durch 1, 2 oder 3 gleiche oder verschiedene Reste aus der Reihe Carboxy, Amino, (C₁-C₄)-Alkylamino, Hydroxy, (C₁-C₄)-Alkoxy, Halogen, Cyano, Di-(C₁-C₄)-alkylamino, Carbamoyl, Sulfamoyl und (C₁-C₄)-Alkoxycarbonyl substituiert ist;
R³ -X-R⁴;
X -O-, -NR⁵-, oder -S-;
R⁴ wie R¹ definiert ist;
wobei R⁴ zusammen mit R² ein Brückenglied -CH₂-CH₂- oder -CH₂-CH₂-CH₂- bilden kann;
R⁵ Wasserstoff oder (C₁-C₆)-Alkyl bedeutet;
A ein anneliertes zyklisches Ringsystem bedeutet, das
a) mit einer, zwei oder drei, vorzugsweise mit einer oder zwei Oxo- oder Hydroxyfunktionen substituiert ist, wobei eine Hydroxy- oder Oxo-Funktion in Nachbarstellung zum Dihydropyranring steht, und
b) ein-, zwei- oder mehrfach, vorzugsweise ein- oder zweifach mit einem (C₁-C₁₀) Alkyl-Rest oder einer Carboxylgruppe substituiert ist, wobei zumindest ein Alkylsubstituent eine funktionelle Gruppe, wie Hydroxy, Carboxy oder Amino trägt;
sowie deren physiologisch verträglichen Salze.

2. Verbindung der Formel I gemäß Anspruch 1, worin bedeuten:
R¹ (C₁-C₈)-Alkyl, (C₀-C₆)-Alkyl-(C₆-C₁₂)-aryl, (C₀-C₆)-Alkyl-(C₃-C₉)-Heteroaryl oder (C₀-C₆)-Alkyl-(C₆-C₁₂)-aryl, wobei der (C₆-C₁₂)-Arylteil mit 1, 2 oder 3 gleichen oder verschiedenen Resten aus der Reihe (C₁-C₆)-Alkyl, Carboxy, Amino, Nitro, Hydroxy, (C₁-C₆)-Alkoxy, Halogen substituiert ist oder zwei benachbarte Reste am (C₆-C₁₂)-Arylring zusammen Methylendioxy bedeuten; bedeutet;
R² Wasserstoff oder (C₁-C₆)-Alkyl, bevorzugt Wasserstoff, bedeutet; .
R³ -X-R⁴ ist;
X -O- , NR5 oder -S- bedeutet;
R ⁴ (C₁-C₆)-Alkyl, das gegebenfalls mit einem oder zwei Resten aus der Reihe OH, NR⁵ substituiert ist; (C₀-C₆)-Alkyl-(C₆-C₁₂)-aryl, vorzugsweise Phenyl oder Benzyl; (C₃-C₆)-Cycloalkyl oder (C₁-C₆)-Alkanoyl bedeutet und
wobei R⁴ zusammen mit R² ein Brückenglied -CH₂-CH₂- oder -CH₂-CH₂-CH₂- bilden kann;
R⁵ Wasserstoff oder (C₁-C₆)-Alkyl bedeutet;
sowie deren physiologisch verträglichen Salze.

3. Verbindung der Formel I gemäß Anspruch 1 - 2, in denen R¹ (C₁-C₆)-Alkyl oder (C₀-C₆)-Alkyl-(C₆-C₁₂)-Aryl bedeutet, sowie deren physiologisch verträglichen Salze.

4. Verbindung der Formel I gemäß einem oder mehreren der Ansprüche 1 bis 3, in denen R² Wasserstoff oder (C₁-C₆)-Alkyl bedeutet, sowie deren physiologisch verträglichen Salze.

5. Verbindung der Formel I gemäß einem oder mehreren der Ansprüche 1 bis 4, in denen X gleich -O- ist, sowie deren physiologisch verträglichen Salze.

6. Verbindung der Formel I gemäß Anspruch 1 bis 5, in denen das annelierte zyklische Ringsystem A Cyclopentan, Cyclohexan , Tetrahydrofuran, Benzol, Pyridin, Imidazol, Pyrazol, Piperazin, Dioxan oder Pyrimidin bedeutet; sowie deren physiologisch verträglichen Salze.

7. Verbindung der Formel I gemäß Anspruch 1 bis 5, in denen das annelierte zyklische Ringsystem A 1,3-Pyrimidin, Benzol, Dioxan oder Cyclohexan bedeutet; sowie deren physiologisch verträglichen Salze.

8. Verbindung der Formel I gemäß einem oder mehreren der Ansprüche 1 - 7 wobei die Verbindung eine Verbindung der Formel 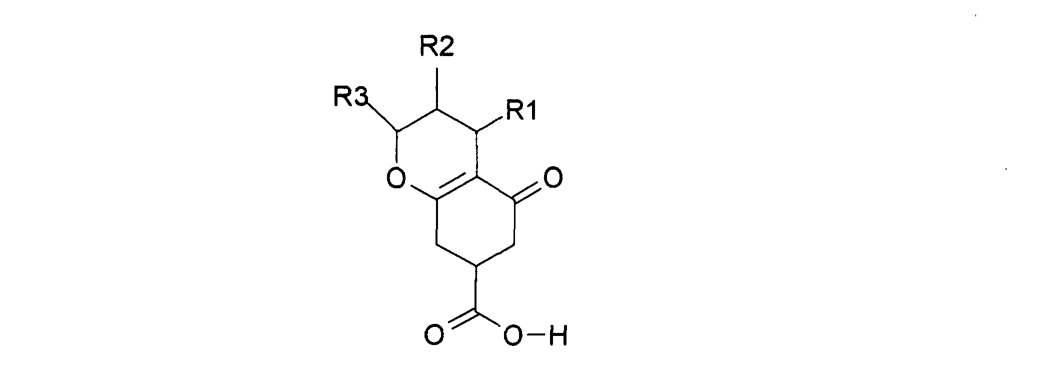 ist; sowie deren physiologisch verträglichen Salze.

9. Verbindung der Formel I gemäß Anspruch 1-8 zur Verwendung als Heilmittel.

10. Arzneimittel enthaltend eine Verbindung nach Anspruch 1 - 8,

## Claims

1. A compound of the formula I 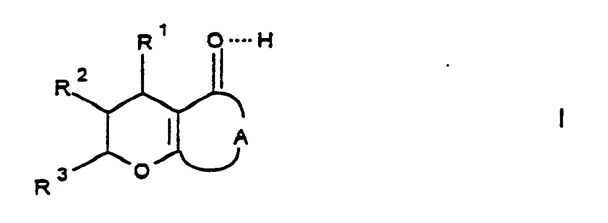 in which:
R¹ is
1. (C₁-C₁₄)-alkyl,
2. (C₂-C₆)-alkenyl,
3. (C₀-C₆)-alkyl-(C₃-C₁₀)-cycloalkyl-(C₀-C₆)-alkyl, where the alkyl moiety is optionally substituted by one or more OH groups,
4. (C₀-C₆)-alkyl-(C₆-C₁₄)-aryl,
5. (C₀-C₆)-alkyl-(C₃-C₉)-heteroaryl,
6. (C₂-C₆)-alkenyl-(C₆-C₁₄)-aryl,
7. (C₂-C₆)-alkenyl-(C₃-C₉)-heteroaryl,
8. (C₁-C₆)-alkanoyl,
9. a radical as defined under 4., 5., 6. or 7., where the (C₆-C₁₄)-aryl or (C₃-C₉)-heteroaryl moiety is substituted by 1, 2 or 3 identical or different radicals from the group consisting of (C₁-C₁₀)-alkyl, carboxyl, amino, nitro, (C₁-C₄)-alkylamino, hydroxyl, (C₁-C₄)-alkoxy, where one to all hydrogen atoms can be replaced by fluorine atoms, (C₆-C₁₂)-aryloxy, halogen, cyano, di-(C₁-C₄)-alkylamino, carbamoyl, sulfamoyl and (C₁-C₄)-alkoxycarbonyl, or two adjacent radicals on the (C₆-C₁₂)-aryl ring together are alkylenedioxy, preferably methylenedioxy;
R² is hydrogen, (C₁-C₁₀)-alkyl, (C₀-C₆)-alkyl-(C₆-C₁₂)-aryl or (C₀-C₆)-alkyl-(C₃-C₉)-heteroaryl, where the C₆-C₁₂-aryl or C₃-C₉-heteroaryl moiety is optionally substituted by 1, 2 or 3 identical or different radicals from the group consisting of carboxyl, amino, (C₁-C₄)-alkylamino, hydroxyl, (C₁-C₄)-alkoxy, halogen, cyano, di-(C₁-C₄)-alkylamino, carbamoyl, sulfamoyl and (C₁-C₄)-alkoxycarbonyl;
R³ is -X-R⁴;
X is -O-, -NR⁵- or -S-;
R⁴ is defined as R¹;
where R⁴ together with R² can form a bridge member -CH₂-CH₂- or -CH₂-CH₂-CH₂-;
R⁵ is hydrogen or (C₁-C₆)-alkyl;
A is a fused cyclic ring system which
a) is substituted by one, two or three, preferably by one or two, oxo or hydroxyl functions, where one hydroxyl or oxo function is in the neighboring position to the dihydropyran ring, and
b) is mono, di- or polysubstituted, preferably mono- or disubstituted, by a (C₁-C₁₀)-alkyl radical or a carboxyl group, where at least one alkyl substituent carries a functional group, such as hydroxyl, carboxyl or amino;
or physiologically tolerable salts thereof.

2. A compound of the formula I as claimed in claim 1, in which:
R¹ is (C₁-C₈)-alkyl, (C₀-C₆)-alkyl-(C₆-C₁₂)-aryl, (C₀-C₆)-alkyl-(C₃-C₉)-heteroaryl or (C₀-C₆)-alkyl-(C₆-C₁₂)-aryl, where the (C₆-C₁₂)-aryl moiety is substituted by 1, 2 or 3 identical or different radicals from the group consiting of (C₁-C₆)-alkyl, carboxyl, amino, nitro, hydroxyl, (C₁-C₆)-alkoxy and halogen or two adjacent radicals on the (C₆-C₁₂)-aryl ring are together methylenedioxy;
R² is hydrogen or (C₁-C₆)-alkyl, preferably hydrogen;
R³ is -X-R⁴;
X is -O-, NR5 or -S-;
R⁴ is (C₁-C₆)-alkyl, which is optionally substituted by one or two radicals from the group consisting of OH and NR⁵; (C₀-C₆)-alkyl-(C₆-C₁₂)-aryl, preferably phenyl or benzyl; (C₃-C₆)-cycloalkyl or (C₁-C₆)-alkanoyl and
where R⁴ together with R² can form a bridge member -CH₂-CH₂- or -CH₂-CH₂-CH₂-;
R⁵ is hydrogen or (C₁-C₆)-alkyl;
or physiologically tolerable salts thereof.

3. A compound of the formula I as claimed in claims 1-2, in which R¹ is (C₁-C₆)-alkyl or (C₀-C₆)-alkyl-(C₆-C₁₂)-aryl, or physiologically tolerable salts thereof.

4. A compound of the formula I as claimed in one or more of claims 1 to 3, in which R² is hydrogen or (C₁-C₆)-alkyl, or physiologically tolerable salts thereof.

5. A compound of the formula I as claimed in one or more of claims 1 to 4, in which X is -O-, or physiologically tolerable salts thereof.

6. A compound of the formula I as claimed in claims 1 to 5, in which the fused cyclic ring system A is cyclopentane, cyclohexane, tetrahydrofuran, benzene, pyridine, imidazole, pyrazole, piperazine, dioxane or pyrimidine; or physiologically tolerable salts thereof.

7. A compound of the formula I as claimed in claims 1 to 5, in which the fused cyclic ring system A is 1,3-pyrimidine, benzene, dioxane or cyclohexane; or physiologically compatible salts thereof.

8. A compound of the formula I as claimed in one or more of claims 1-7, where the compound is a compound of the formula 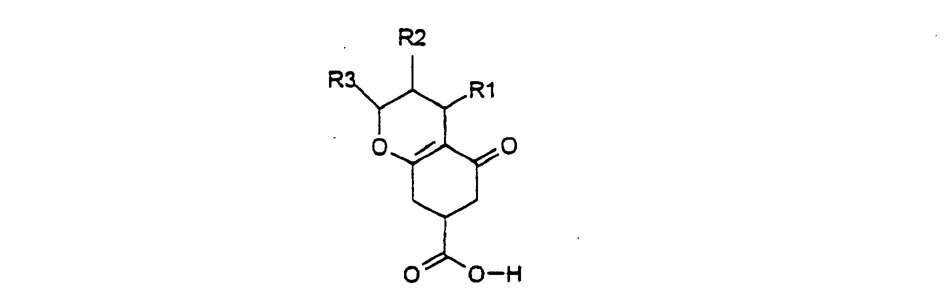 or physiologically tolerable salts thereof.

9. A compound of the formula I as claimed in claims 1 to 8 for use as a therapeutic.

10. A pharmaceutical comprising a compound as claimed in claims 1 to 8.

## Revendications

1. Composés de formule I 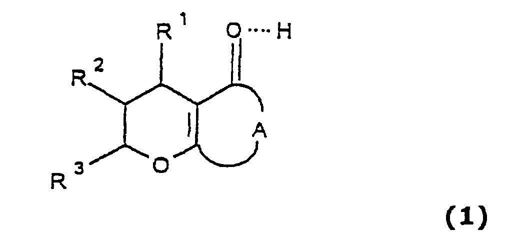 dans laquelle
R¹ représente des groupes
1. alkyle en C₁-C₁₄,
2. alcényle en C₂-C₆,
3. (alkyl en C₀-C₆) - (cycloalkyl en C₃-C₁₀)-(alkyle en C₀-C₆), le fragment alkyle étant éventuellement substitué par un ou plusieurs groupes OH,
4. (alkyl en C₀-C₆)-(aryle en C₆-C₁₄),
5. (alkyl en C₀-C₆)-(hétéroaryle en C₃-C₉),
6. (alcényl en C₂-C₆)-(aryle en C₆-C₁₄),
7. (alcényl en C₂-C₆)-(hétéroaryle en C₃-C₉),
8. alcanoyle en C₁-C₆,
9. un reste tel que défini au points 4., 5., 6. ou 7., le fragment aryle en C₆-C₁₄ ou hétéroaryle en C₃-C₉ pouvant être substitués par 1, 2 ou 3 restes identiques ou différents pris dans le groupe comprenant alkyle en C₁-C₁₀, carboxy, amino, nitro, (alkyl en C₁-C₄)amino, hydroxy, alkoxy en C₁-C₄, un à tous les atomes d'hydrogène pouvant être remplacés par des atomes de fluor, aryloxy en C₆-C₁₂, halogène, cyano, di-(alkyl en C₁-C₄)-amino, carbamoyle, sulfamoyle et (alkoxy en C₁-C₄)carbonyle, ou deux des restes voisins sur le cycle aryle en C₆-C₁₂ représentent conjointement un groupe alkylènedioxy, de préférence méthylènedioxy ;
R² représente un atome d'hydrogène, des groupes alkyle en C₁-C₁₀, (alkyl en C₀-C₆)-(aryle en C₆-C₁₂), (alkyl en C₀-C₆) - (hétéroaryle en C₃-C₉), le fragment aryle C₆-C₁₂ ou hétéroaryle en C₃-C₉ étant éventuellement substitué par 1, 2 ou 3 restes identiques ou différents pris dans le groupe comprenant carboxy, amino, (alkyl en C₁-C₄)-amino, hydroxy, alkoxy en C₁-C₄, halogène, cyano, di-(alkyl en C₁-C₄) amino, carbamoyle, sulfamoyle et (alkoxy en C₁-C₄)carbonyle ;
R³ représente un groupe -X-R⁴ ;
X représente des groupes -O-, -NR⁵- ou -S- ;
R⁴ est défini comme R¹ ;
R⁴ peut former conjointement avec R² un élément de pontage -CH₂-CH₂- ou -CH₂-CH₂-CH₂- ;
R⁵ représente un atome d'hydrogène ou un groupe alkyle en C₁-C₆ ;
A représente un système cyclique de cycles condensés qui
a) est substitué par une, deux ou trois, de préférence par une ou deux fonctions oxo ou hydroxy, une fonction hydroxy ou oxo pouvant se trouver au voisinage du cycle dihydropyrane, et
b) est substitué une, deux ou plusieurs fois, de préférence une ou deux fois par un reste alkyle en C₁-C₁₀ ou un groupe carboxyle, au moins un substituant alkyle porte un groupe fonctionnel, tel que hydroxy, carboxy ou amino ;
ainsi que leurs sels physiologiquement tolérés.

2. Composés de formule I selon la revendication 1, où
R¹ représente des groupes alkyle en C₁-C₈, (alkyl en C₀-C₆) - (aryle en C₆-C₁₂), (alkyl en C₀-C₆)-(hétéroaryle en C₃-C₉) ou (alkyl en C₀-C₆) - (aryle en C₆-C₁₂), le fragment aryle en C₆-C₁₂ pouvant être substitué par 1, 2 ou 3 restes identiques ou différents pris dans le groupe comprenant alkyle en C₁-C₆, carboxy, amino, nitro, hydroxy, alkoxy en C₁-C₆, halogène, ou deux restes voisins sur le cycle aryle en C₆-C₁₂ représentent conjointement un groupe méthylènedioxy ;
R² représente un atome d'hydrogène ou un groupe alkyle en C₁-C₆ de préférence un atome d'hydrogène ;
R³ représente un groupe -X-R⁴ ;
X représente des groupes -O-, -NR⁵- ou -S- ;
R⁴ représente un groupe alkyl en C₁-C₆ éventuellement substitué par un ou deux restes pris dans le groupe comprenant OH, NR⁵ ; un groupe (alkyl en C₀-C₆)-(aryle en C₆-C₁₂), de préférence phényle ou benzyle ; cycloalkyle en C₃-C₆ ou alcanoyle en C₁-C₆ et
R⁴ pouvant former conjointement avec R² un élément de pontage -CH₂-CH₂- ou -CH₂-CH₂-CH₂- ;
R⁵ représente un atome d'hydrogène ou un groupe alkyle en C₁-C₆ ;
ainsi que leurs sels physiologiquement tolérés.

3. Composés de formule I selon la revendication 1-2, où R¹ représente un groupe alkyle en C₁-C₆ ou (alkyl en C₀-C₆) - (aryle en C₆-C₁₂), ainsi que leurs sels physiologiquement tolérés.

4. Composés de formule I selon une ou plusieurs des revendications 1 à 3, où R² représente un atome d'hydrogène ou un groupe alkyle en C₁-C₆, ainsi que leurs sels physiologiquement tolérés.

5. Composés de formule I selon une ou plusieurs des revendications 1 à 4, où X représente -O- ainsi que leurs sels physiologiquement tolérés.

6. Composés de formule I selon la revendication 1 à 5, où le systèmes cyclique de cycles condensés A représente cyclopentane, cyclohexane, tétrahydrofuranne, benzène, pyridine, imidazole, pyrazole, pipérazine, dioxanne ou pyrimidine, ainsi que leurs sels physiologiquement tolérés.

7. Composés de formule I selon la revendication 1 à 5, où le système cyclique de cycles condensés A représente des groupes 1,3-pyrimidine, benzène, dioxanne ou cyclohexane ; ainsi que leurs sels physiologiquement tolérés.

8. Composés de formule I selon une ou plusieurs des revendications 1 à 7, où le composé représente un composé de formule 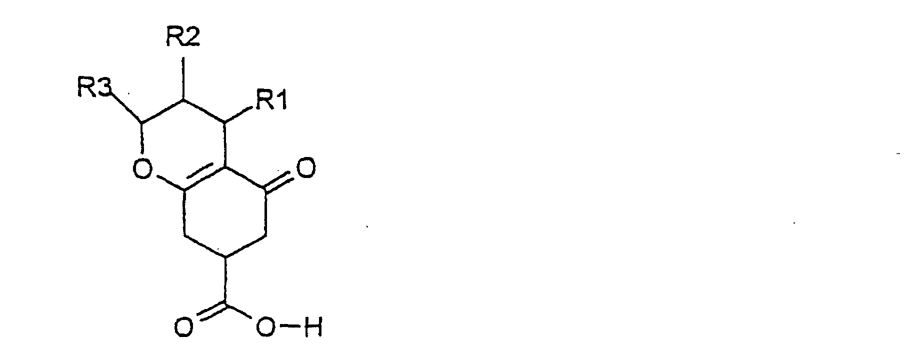 ainsi que leurs sels physiologiquement tolérés.

9. Composés de formule I selon la revendication 1 à 8 pour l'utilisation comme médicament.

10. Médicament renfermant un composé selon la revendication 1 à 8.
